# EUROPEAN PATENT SPECIFICATION

(11) **EP 1 173 105 B1**
(45) Date of publication and mention of the grant of the patent: **27.10.2004**
(21) Application number: 00923554.0
(22) Date of filing: 22.04.2000
(51) Int. Cl.: A61B 19/00

(54) **APPARATUS AND METHOD FOR IMAGE GUIDED SURGERY**
VORRICHTUNG UND VERFAHREN FÜR BILDGESTEUERTE CHIRURGIE
APPAREIL ET PROCEDE DE CHIRURGIE GUIDEE PAR L'IMAGERIE

(30) Priority: 22.04.1999 US 295591
(43) Date of publication of application: 23.01.2002
(60) Divisional application: 04021113.8
(73) Proprietor: Medtronic Surgical Navigation Technologies, Louisville, CO 80027 (US)
(72) Inventor: MELKENT, Anthony, J., Lafayette, CO 80026 (US); CARLS, Thomas, A., Memphis, TN 38103 (US); SIMON, David, A., Boulder, CO 80303 (US); FOLEY, Kevin, Germantown, TN 38139 (US)
(74) Representative: Luckhurst, Anthony Henry William
(86) International application number: PCT/US2000/010712
(87) International publication number: WO 2000/064367

(56) References cited:
- WO-A-96/11624
- US-A- 5 622 170
- US-A- 5 695 501
- US-A- 5 776 064
- US-A- 5 799 055
- US-A- 5 848 967
- US-A- 5 868 675

## Description

The present invention is directed generally to image guided surgery and, more particularly, to apparatus and methods of using image guided surgery for landmark-dependent surgical procedures, such as interbody fusion device placement.

### Description of the Related Art:

Many surgical procedures require the surgeon to establish with some accuracy a "landmark" on the patients anatomy as a point of reference for aspects of the procedure. This is particularly the case, for example, in spinal surgery requiring the implantation of interbody fusion devices, including cage-type and bone dowel-type devices, where the desired implantation location is referenced to a hypothetical plane oriented with respect to the spinal column, termed the "midline plane." The surgeon typically will use this plane as a reference for the implantation location of the fusion device, and the manufacturers of these devices often specify the preferred implantation locations with reference to this landmark or figure.

Current practice for interbody fusion device placement involves determining a midline plane of the vertebral column by taking multiple fluoroscopic images. Once this is determined, a midline is marked with an ink pen or equivalent directly on the vertebral body and/or disc of the patient and is used by the surgeon to determine the "offset," that is, the position to the right or left of the midline on which the interbody fusion device will be centered. This is usually accomplished by placing instruments, which have built-in offsets, dependent upon the gauging size of the fusion devices, on this point, and the interbody fusion device offset location is marked with the pen.

The surgeon then has to mentally construct the midline plane through the marked midline in order to establish the trajectory passing through the offset position. This can be difficult to do and dependent on careful patient positioning (i.e., if the patient is not lying comptetety flat on the table, the surgeon may induce this rotational error into his mental image of the midline plane). The fusion device implantation instrumentation is then manually positioned on the marked offset location. Instrument trajectory orientation is accomplished using multiple fluoroscopic views to be taken. This may subject the patient, the surgeon, and the operating room personnel to excessive radiation exposure.

Techniques are known through which x-ray images are used to locate the real-time position of surgical instruments in the patient anatomy represented by the x-ray image without requiring x-rays to be repeatedly taken. By tracking the instrument, the real-time position of the instrument in anatomy represented by the x-ray image is determined, and a corresponding representation of the instrument in the x-ray image is displayed. This allows the surgeon to continually observe the progress of the surgery without necessitating additional x-ray images, thereby reducing the total exposure to the patient and operating room personnel.

US Patent Number 5,868,675 discloses an intracranial surgical system that images and displays a 3D representation of a patient's skull. The disclosure relates to optimising the transfer of reference frames, by reducing to a minimum the deviations between the coordinates of the image of the patient on the screen and base points on the actual patient. The base points can be special marks affixed to the patient or notable points on the patient.

### SUMMARY OF THE INVENTION

It is the object of the present invention to eliminate the need for a surgeon to mark anatomical landmarks, such as the midline plane, directly on the patient.

It is also an object of the present invention to reduce the number of images, and thus amount of radiation and OR time, needed to properly locate instruments relative to a landmark.

It is a further object to reduce the error in positioning surgical instruments relative to such anatomical landmarks.

It is a still further object to provide near real-time instrument position information relative to such anatomical landmarks.

It is yet another object to utilize, and enhance the capabilities of, image guided surgical systems such as are described in the afore-mentioned related application, for interbody fusion device placement.

In accordance with the present invention, as embodied and broadly described herein, an apparatus is provided for use with an image-based surgical system to display anatomical landmarks superimposed on an image of the anatomy to be operated upon. In one aspect, the present invention provides apparatus for use in an interbody fusion device placement surgical procedure, the apparatus for use with an image guided surgical system, the system including a computer processor, a display device coupled to the processor, and a memory associated with the processor and having stored computer instructions that, when executed, cause the processor to display on the display device at least an A-P image or a lateral image corresponding to the portion of a spine on which the placement procedure is to be performed (2082), the apparatus characterised by comprising an interface (2010, 2020) for inputting one or more positions to the processor and for generating a midline plane landmark; additional computer instructions stored in the memory that, when executed, cause the processor to generate and display on the display device in registration with at least one of the displayed A-P and lateral images, a midline plane corresponding to said one or more positions; and further instructions for generating offset implantation positions relative to said midline plane based on inputted offset position values. The interface can be selected from a keyboard, a mouse, or a light pen. Where the surgical system is an image guided surgical system, the interface includes a trackable surgical probe. Also, where the surgical system is an image guided surgical system having one or more trackable instruments, the additional instructions include instructions that, when executed, cause the processor to generate and display an icon indicating the relative position and orientation of a tracked instrument relative to the anatomical landmark or to a location referenced to the landmark. The processor can also overlay iconic representations of the instruments on the displayed image and landmark.

Where the system is an image guided surgery system, the inputting step can include applying a tracked surgical probe to the anatomy to be operated upon. Also, where the system is an image guided surgery system having one or more trackable surgical instruments, the memory includes instructions that, when executed, cause the processor to generate and display on the display device, an icon representating the relative position and orientation of a tracked instrument relative to the displayed anatomical landmark or to a location referenced to the landmark. The processor also can generate and display iconic representations of the instruments overlayed in registration with the displayed image and landmark.

### BRIEF DESCRIPTION OF THE DRAWINGS

The accompanying drawings, which are incorporated in and constitute a part of this specification, illustrate several embodiments consistent with this invention and, together with the description, help explain the principles of the invention. In the drawings,
Fig. 1 is a diagram of apparatus in accordance with the present invention in use with an image guided surgery system;
Figs. 2A and 2B are schematic x-ray images illustrating true and distorted images, respectively;
Figs. 3A and 3B illustrate a projective transformation in a fluoroscopic C-arm imager;
Fig. 4 is a flow chart illustrating methods for performing two-dimensional navigational guidance using the imaging system;
Figs. 5A and 5B are exemplary fluoroscopic x-ray images illustrating the iconic graphical overlay of a surgical instrument;
Fig. 6 is a fluoroscopic image including a "cross hair" graphical overlay of an instrument;
Figs. 7A-7C illustrate images of complementary views and an axis that relates them;
Fig. 8 is an image of a lateral view of a patient's vertebral disc;
Fig. 9 is an image of a lateral view of a spinal vertebra;
Fig. 10 is a diagram illustrating a system for specifying a planned trajectory of a surgical instrument;
Fig. 11 is a flow chart illustrating a method for specifying a planned trajectory of a surgical instrument;
Figs. 12A through 12C are images of a fracture of a femur containing two bone fragments;
Fig. 13 is a flow chart illustrating methods for aligning bone fragments using the imaging system;
Figs 14A and 14B are images illustrating implantation of an inter-vertebral case in the spine of a patient;
Figs. 15A through 15C are images used in the acquisition of an x-ray view of the medial axis of a vertebral pedicle;
Figs. 16A and 16B are images used to illustrate the measurement of out-of-plane angles based on fluoroscopic images.
Fig. 17A shows actual apparatus embodying the present invention for interbody fusion device placement, and Figs. 17B and 17C show corresponding A-P and lateral x-ray images obtained by the image guided surgery system used by the apparatus of Fig. 17A;
Figs. 18A and 18B correspond to the A-P image in Fig. 17B showing two inputted anatomical landmark positions and the midline plane generated from the inputted positions, respectively, displayed in registration with the image;
Fig. 19A shows a tracked surgical probe for inputting anatomical landmark positions directly from the anatomy of a patient, and Fig. 19B shows a midline plane generated from the positions inputted in Fig. 19A displayed in registration with the A-P image;
Figs. 20A-20E are schematic drawings of trackable instruments usable with the apparatus of Fig. 17A namely, distractor, working channel, reamer, tap, and implant inserter, respectively;
Figs. 21A and 21 B through Figs. 25A and 25B correspond to the A-P and lateral x-ray images of Figs. 17B and 17C with icons representing the distractor position in registration with the image and an icon representing relative distance from the offset position, during successive times in the distractor placement step;
Figs. 26A and 26B through Figs. 29A and 29B correspond to the A-P and lateral x-ray images of Figs. 17B and 17C with icons representing the working channel position in registration with the image and an icon representing relative distance from the offset position, during a succession of times for a working channel placement step;
Figs. 30A and 30B through Figs. 34A and 34B correspond to A-P and lateral x-ray images of Figs. 17B and 17C with icons representing the respective instruments position in registration with the image and an icon representing relative distance from the offset position; during the reaming, tapping, and interbody fusion device placement steps, and
Fig. 35 is a flow chart of an interbody fusion device placement procedure using a method in accordance with the present invention.

Reference will now be made in detail to embodiments of the invention, examples of which are illustrated in the accompanying drawings. Wherever possible, the same reference numbers will be used throughout the drawings to refer to the same or like parts.

### DETAILED DESCRIPTION

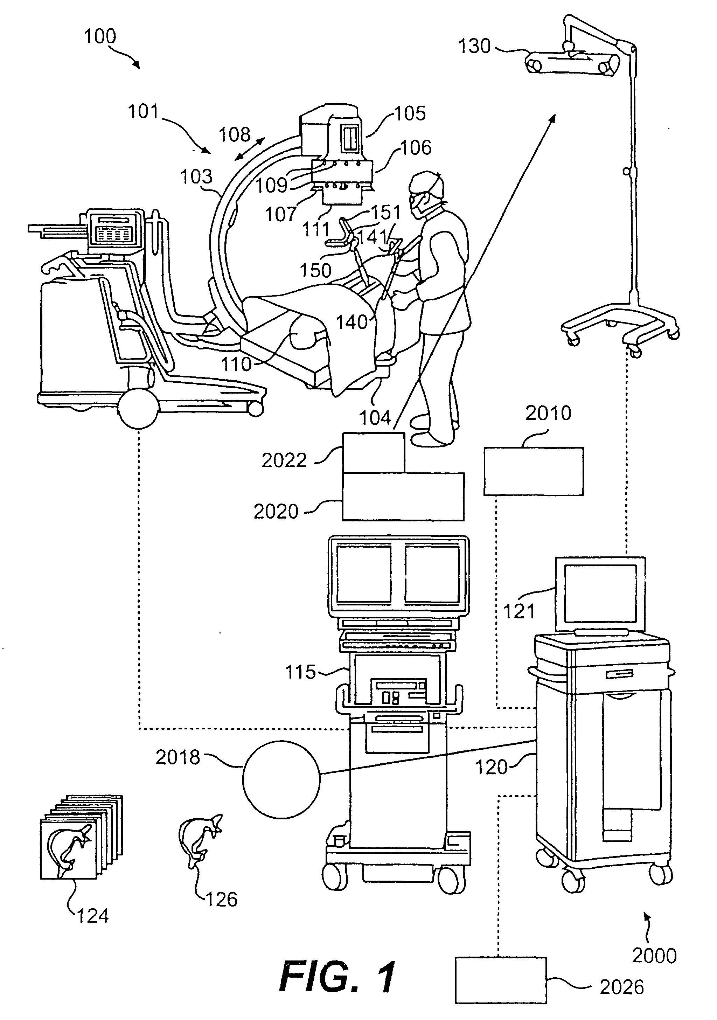

With initial reference to Fig. 1, there is shown schematically apparatus made in accordance with the present invention for image guided interbody fusion device placement and designated generally by the numeral 2000. Apparatus 2000 enables a surgeon using an image-based surgical system to generate and display on a monitor or other display device anatomical landmarks overlayed or superimposed in registration with the display of an acquired or preacquired image provided by the surgical system, of the portion of the patients' vertebral column on which the fusion body placement procedure is to be performed. Used in conjunction with an image guided surgery system that can provide real time indications of the positioning of "tracked" surgical instruments, apparatus 2000 also allows the surgeon to position the instruments relative to the displayed landmark (in this case, a midline plane) or a location referenced to the midline (e.g., an offset position for fusion device placement surgery) as well as to the displayed true image. While the present invention to be described in more detail below is exemplified by a system used for interbody fusion device placement, it is not so limited.

### IMAGE GUIDED SYSTEM OVERVIEW

Apparatus 2000 is shown in Fig. 1 in use with a preferred image guided surgical system 100. System 100, to be described henceforth in sufficient detail to allow an understanding and appreciation of the present invention, is explained in greater detail in U.S. Patent Application No. 09/274,972 of David A. Simon et al., filed March 23, 1999 and entitled "Navigational Guidance Via Computer - Assisted Fluoroscopic Imaging,". However, it must be understood that the invention is not confined to use with this particular image guided surgical system.

Schematically shown in Fig. 1 is a diagram of an imaging-based system 100 used to acquire and display x-ray images appropriate for the surgical procedure, such as anterior-posterior ("A-P") and laterai images of a spine section for an interbody fusing device placement procedure. Imaging system 100 includes a fluoroscopic C-arm x-ray imaging device 101 that further includes C-arm 103, x-ray source 104, x-ray receiving section 105, a calibration and tracking target 106, and radiation sensors 107. Calibration and tracking target 106 includes infrared reflectors (or alternatively infrared emitters) 109 and calibration markers 111. System 100 includes C-arm control computer 115 which allows a physician to control the operation of imaging device 101, such as setting imaging parameters.

One appropriate implementation of imaging device 101 is the "Series 9600 Mobile Digital Imaging System," from OEC Medical Systems, Inc., of Salt Lake City, Utah, although calibration and tracking target 106 and radiation sensors 107 are typically not included in the Series 9600 Mobile Digital Imaging System and may have to be added. The "Series 9600 Mobile Digital Imaging System" is otherwise structurally similar to imaging system 100.

In operation, x-ray source 104 generates x-rays that propagate through patient 110 and calibration target 106, and into x-ray receiving section 105. Receiving section 105 generates an image representing the intensities of the received x-rays. Typically, receiving section 105 comprises an image intensifier that converts the x-rays to visible light and a charge coupled device (CCD) video camera that converts the visible light to digital images. Receiving section 105 may also be a device that converts x-rays directly to digital images, thus potentially avoiding distortion introduced by first converting to visible light.

Fluoroscopic images taken by imaging device 101 are transmitted to computer 115 where they may further be forwarded to computer 120. Computer 120 provides facilities for displaying (on monitor 121) saving, digitally manipulating, or printing a hard copy of the received images. Three-dimensional images, such as pre-acquired patient specific CT/MR data set 124 or a, three-dimensional atlas data set 126 (described in more detail below) may also be manipulated by computer 120 and displayed by monitor 121. Images, instead of or in addition to being displayed on monitor 121, may also be displayed to the physician through a heads-up-display.

Although computers 115 and 120 are shown as two separate computers, they alternatively could be variously implemented as multiple computers or as a single computer that performs the functions performed by computers 115 and 120. In this case, the single computer would receive input from both C-arm imager 100 and tracking sensor 130.

Radiation sensors 107 sense the presence of radiation, which is used to determine whether or not imaging device 100 is actively imaging. The result of their detection is transmitted to processing computer 120. Alternatively, a person may manually indicate when device 100 is actively imaging or this function can be built into x-ray source 104, x-ray receiving section 105, or control computer 115.

In operation, the patient is positioned between the x-ray source 104 and the x-ray receiving section 105. In response to an operator's command input to control computer 115, x-rays emanate from source 104 and pass through patient 110, calibration target 106, and into receiving section 105, which generates a two-dimensional image of the patient.

C-arm 103 is capable of rotating relative to patient 110, allowing images of patient 110 to be taken from multiple directions. For example, the physician may rotate C-arm 103 ) in the direction of arrows 108 or about the long axis of the patient. Each of these directions of movement involves rotation about a mechanical axis of the C-arm. In this example, the long axis of the patient is aligned with the mechanical axis of the C-arm.

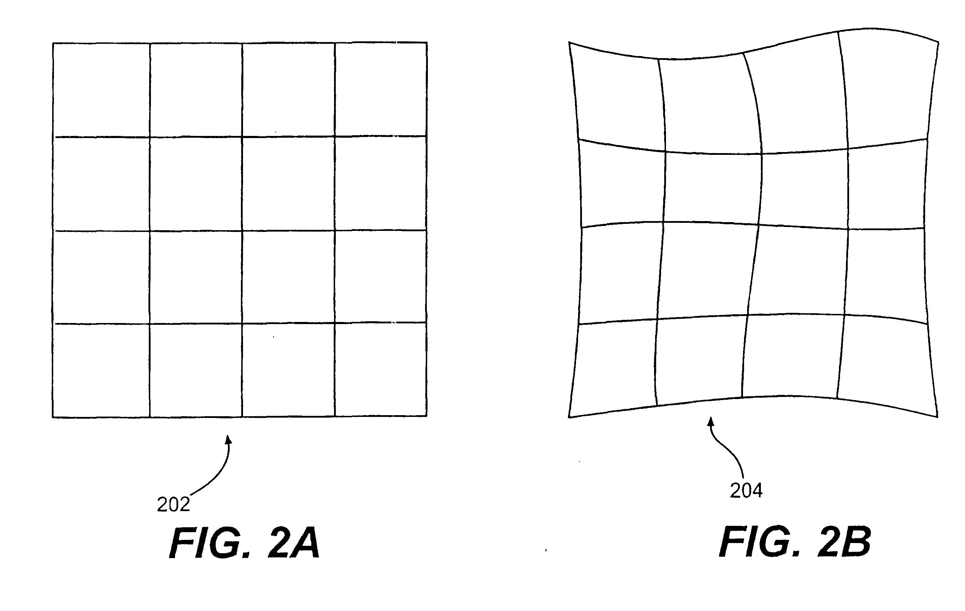

Raw images generated by receiving section 105 tend to suffer from undesirable distortion caused by a number of factors, including inherent image distortion in the image intensifier and external electromagnetic fields. Drawings representing ideal and distorted images are shown in Fig. 2. Checkerboard 202 represents the ideal image of a checkerboard shaped object. The image taken by receiving section 105, however, can suffer significant distortion, as illustrated by distorted image 204.

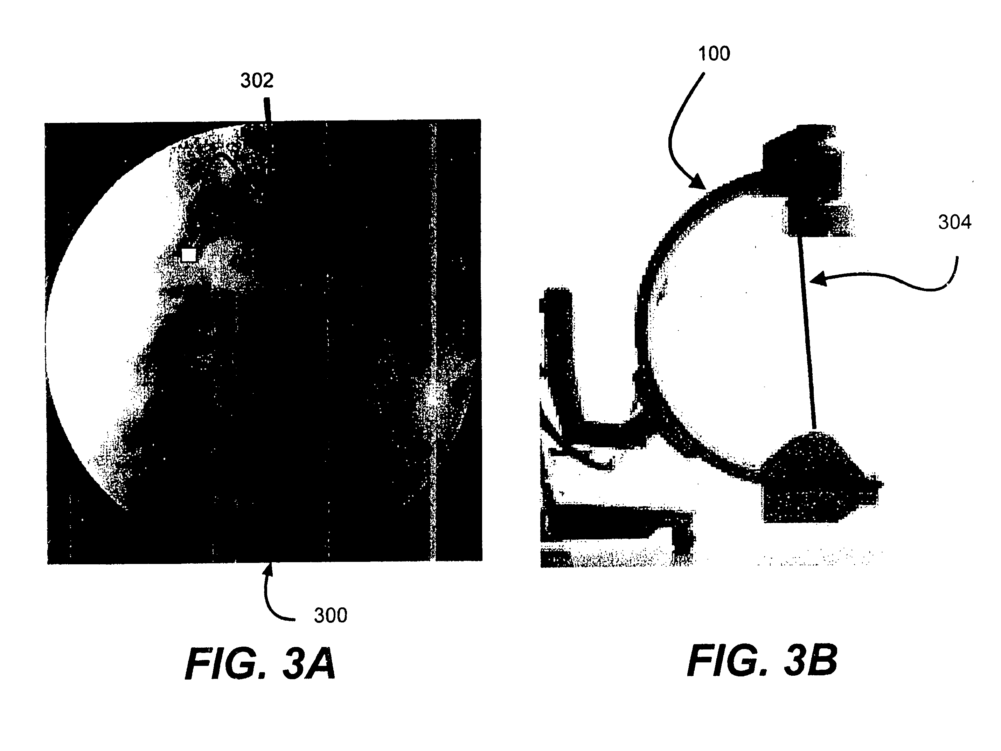

The image formation process in a system such as fluoroscopic C-arm imager 100 is governed by a geometric projective transformation which maps lines in the fluoroscope's field of view to points in the image (i.e., within the x-ray receiving section 105). This concept is illustrated in Figs. 3A and 3B. Image 300 (and any image generated by the fluoroscope) is composed of discrete picture elements (pixels), an example of which is labeled as 302. Every pixel within image 300 has a corresponding three-dimensional line in the fluoroscope's field of view. For example, the line corresponding to pixel 302 is labeled as 304. The complete mapping between image pixels and corresponding lines governs projection of objects within the field of view into the image. The intensity value at pixel 302 is determined by the densities of the object elements (i.e., portions of a patient's anatomy, operating room table, etc.) intersected by the line 304. For the purposes of computer assisted navigational guidance, it is necessary to estimate the projective transformation which maps lines in the field of view to pixels in the image, and vice versa. Geometric projective transformation is well known in the art.

Intrinsic calibration, which is the process of correcting image distortion in a received image and establishing the projective transformation for that image, involves placing "calibration markers" in the path of the x-ray, where a calibration marker is an object opaque or semi-opaque to x-rays. Calibration markers 111 are rigidly arranged in predetermined patterns in one or more planes in the path of the x-rays and are visible in the recorded images. Tracking targets, such as emitters or reflectors 109, are fixed in a rigid and known position relative to calibration markers 111.

Because the true relative position of the calibration markers 111 in the recorded images are known, computer 120 is able to calculate an amount of distortion at each pixel in the image (where a pixel is a single point in the image). Accordingly, computer 120 can digitally compensate for the distortion in the image and generate a distortion-free, or at least a distortion improved image. While not preferred, distortion may be left in the image, and subsequent operations on the image, such as superimposing an iconic representation of a surgical instrument on the image (described in more detail below), may be distorted to match the image distortion determined by the calibration markers. The same calibration markers can also be used to estimate the geometric perspective transformation, since the position of these markers are known with respect to the tracking target emitters or reflectors 109 and ultimately with respect to tracking sensor 130. A more detailed explanation of methods for performing intrinsic calibration is described in the references B. Schuele et al., "Correction of Image Intensifier Distortion for Three-Dimensional Reconstruction," presented at SPIE Medical Imaging 1995. San Diego, CA, 1995 and G. Champleboux et al., "Accurate Calibration of Cameras and Range Imaging Sensors: the NPBS Method," Proceedings of the 1992 IEEE International Conference on Robotics and Automation, Nice, France, May 1992, and U.S. Application Serial Number 09/106, 109, filed on June 29, 1998 by the present assignee.

Calibration and tracking target 106 may be attached to x-ray receiving section 105 of the C-arm. Alternately, the target 106 can be mechanically independent of the C-arm, in which case it should be positioned such that the included calibration markers 111 are visible in each fluoroscopic image to be used in navigational guidance. Element 106 serves two functions. The first, as described above, is holding calibration markers 111 used in intrinsic calibration. The second function, which is described in more detail below, is holding infrared emitters or reflectors 109, which act as a tracking target for tracking sensor 130. Tracking sensor 130 is a real-time infrared tracking sensor linked to computer 120. Specially constructed surgical instruments and other markers in the field of tracking sensor 130 can be detected and located in three-dimensional space. For example, a surgical instrument 140, such as a drill, is embedded with infrared emitters or reflectors 141 on its handle. Tracking sensor 130 detects the presence and location of infrared emitters or reflectors 141. Because the relative spatial locations of the emitters or reflectors in instrument 140 are known, tracking sensor 130 and computer 120 are able to locate instrument 140 in three-dimensional space using well known mathematical transformations. Instead of using infrared tracking sensor 130 and corresponding infrared emitters or reflectors, other types of positional location devices are known in the art, and may be used. For example, a positional location device may also be based on magnetic fields, sonic emissions, or radio waves.

Reference frame marker 150, like surgical instrument 140, is embedded with infrared emitters or reflectors, labeled 151. As with instrument 140, tracking sensor 130 similarly detects the spatial location of emitters/reflectors 151, through which tracking sensor 130 and computer 120 determine the three-dimensional position of dynamic reference frame marker 150. The determination of the three-dimensional position of an object relative to a patient is known in the art, and is discussed, for example, in the following references: PCT Publication WO 96/11624 to Bucholz et al., published April 25, 1996; U.S. Patent No. 5,384,454 to Bucholz; U.S. Patent No. 5,851,183 to Bucholz, and U.S. Patent No. 5,871,445 to Bucholz.

During an operation, dynamic reference frame marker 150 is attached in a fixed position relative to the portion of the patient to be operated on. For example, when inserting a screw into the spine of patient 110, dynamic reference frame marker 150 may be physically attached to a portion of the spine of the patient. Because dynamic reference frame 150 is in a fixed position relative to the patient anatomy, and instrument 140 can be accurately located in three dimensional space relative to dynamic reference frame 150, instrument 140 can also be located relative to the patient's anatomy.

As discussed above, calibration and tracking target 106 also includes infrared emitters or reflectors 109 similar to those in instrument 140 or dynamic reference frame 150. Accordingly, tracking sensor 130 and computer 120 may determine the three-dimensional position of calibration target 106 relative to instrument 140 and/or dynamic reference frame 150 and thus the patient position.

In general, the imaging system 100 shown in Fig. 1 assists physicians performing surgery by displaying real-time or pre-acquired images, such as fluoroscopic x-ray images, of patient 110 on display 121. Representations of surgical instruments 140 are overlaid on pre-acquired fluoroscopic images of patient 110 based on the position of the instruments determined by tracking sensor 130. In this manner, the physician is able to see the location of the instrument relative to the patient's anatomy, without the need to acquire real-time fluoroscopic images, thus greatly reducing radiation exposure to the patient and to the surgical team. "Pre-acquired," as used herein, is not intended to imply any required minimum duration between receipt of the x-ray signals and displaying the corresponding image. Momentarily storing the corresponding digital signal in computer memory while displaying the fluoroscopic image constitutes preacquiring the image.

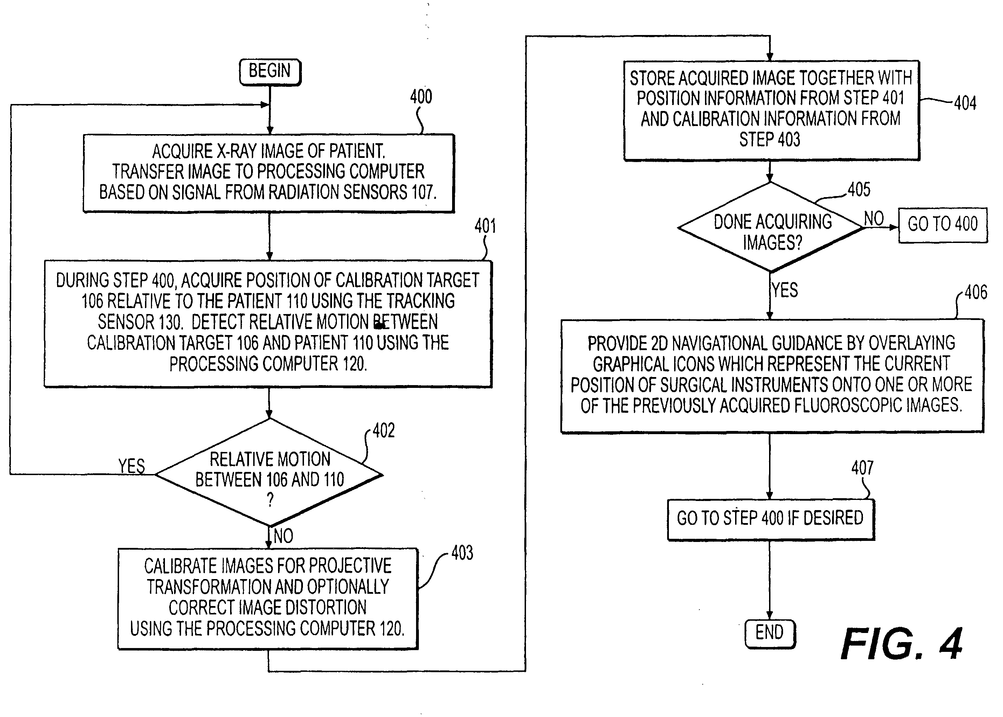

Fig. 4 is a flow chart illustrating methods for performing two-dimensional navigational guidance using system 100 of Fig. 1. The physician begins by acquiring one or more fluoroscopic x-ray images of patient 110 using imager 101 (step 400). As previously mentioned, acquiring an x-ray image triggers radiation sensors 107, which informs computer 120 of the beginning and end of the radiation cycle used to generate the image. For a fluoroscopic x-ray image acquired with imager 101 to be useable for navigational guidance imager 101, when acquiring the image, should be stationary with respect to patient 110. If C-arm 103 or patient 110 is moving during image acquisition, the position of the fluoroscope will not be accurately determined relative to the patient's reference frame. Thus, it is important that the recorded position of imager 101 reflects the true position of the imager at the time of image acquisition. If imager 101 moves during the image acquisition process, or if imager 101 moves after image acquisition but before its position is recorded, the calibration will be erroneous, thus resulting in incorrect graphical overlays. To prevent this type of erroneous image, computer 120 may examine the position information from tracking sensor 130 while radiation sensors 107 are signaling radiation detection. If the calibration and tracking target 106 moves relative to dynamic reference frame 150 during image acquisition, this image is marked as erroneous. (Steps 401 and 402).

At the end of the radiation cycle, computer 120 retrieves the acquired image from C-arm control computer 115 and retrieves the location information of target marker 106 and dynamic reference frame 150 from tracking sensor 130. Computer 120 calibrates the acquired image, as described above, to team its projective transformation and optionally to correct distortion in the image, (step 403), and then stores the image along with its positional information (step 404). The process of steps 400-404 is repeated for each image that is to be acquired (step 405).

Because the acquired images are stored with the positional information of the calibration and tracking target 106 and dynamic reference frame 150, the position of C-arm 103, x-ray source 104, and receiving section 105 for each image, relative to patient 110, can be computed based upon the projective transformation identified in the calibration process.

During surgery, tracking sensor 130 and computer 120 detect the position of instrument 140 relative to dynamic reference frame 150, and hence relative to patient 110. With this information, computer 120 dynamically calculates, in real-time, the projection of instrument 140 into each fluoroscopic image as the instrument is moved by the physician. A graphical representation of instrument 140 may then be overlaid on the fluoroscopic images (step 406). The graphical representation of instrument 140 is an iconic representation of where the actual surgical instrument would appear within the acquired fluoroscopic x-ray image if imager 101 was continuously acquiring new images from the same view as the original image. There is no theoretical limit to the number of fluoroscopic images on which the graphical representations of instrument 140 may be simultaneously overlaid.

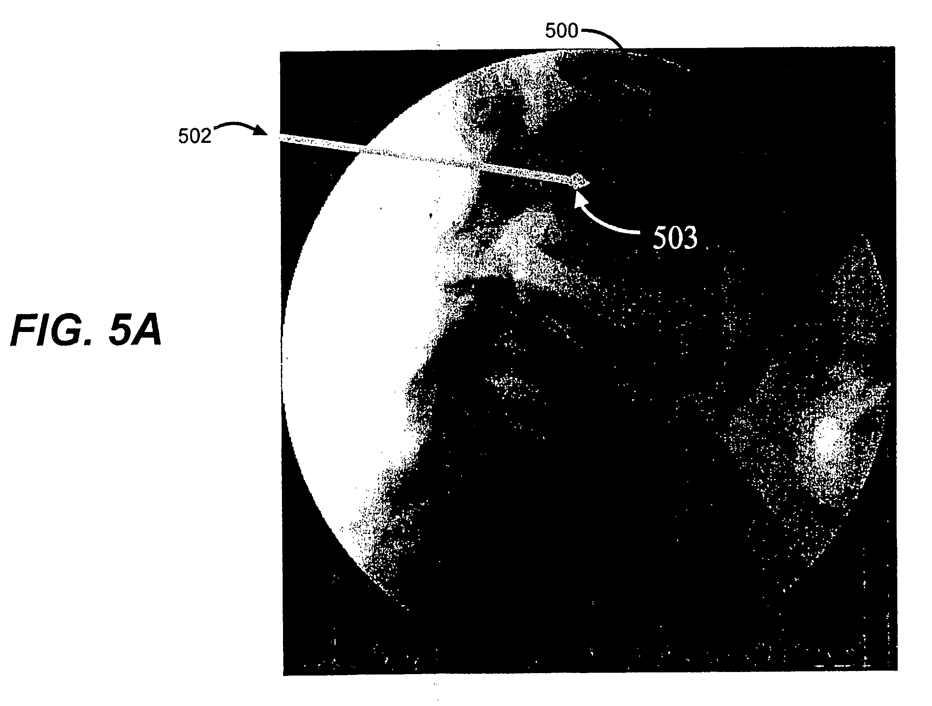

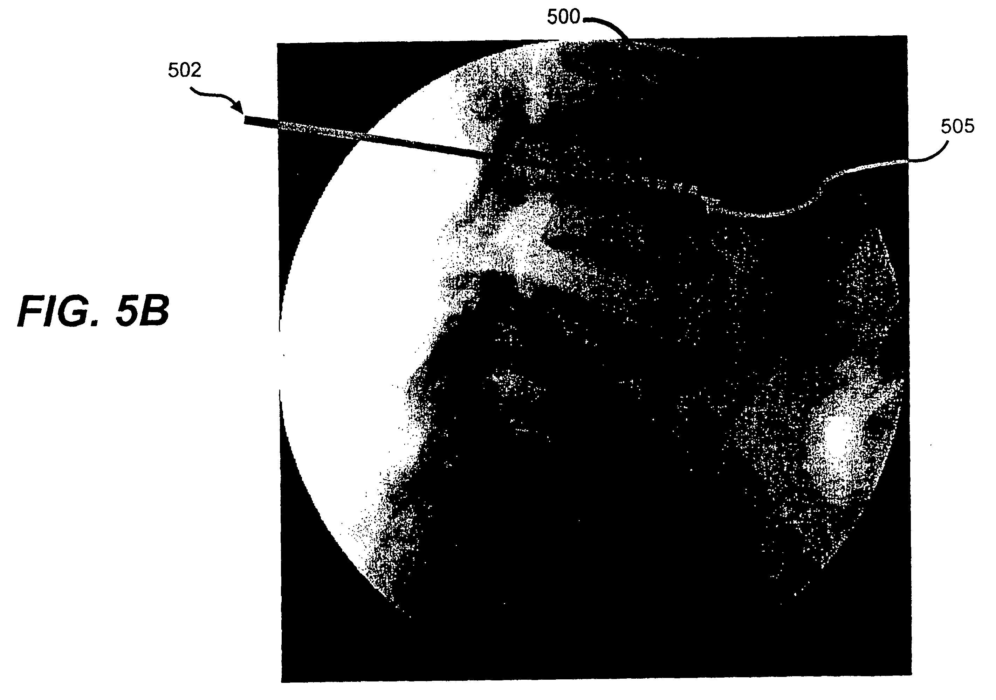

Figs. 5A and 5B are exemplary fluoroscopic x-ray images illustrating the iconic graphical overlay of a surgical instrument. Fluoroscopic image 500, shown in Fig. 5A, is an image of a lateral view of the lumbar spine. Graphical overlay 502 is the iconic overlay of a surgical instrument, such as a drill, within image 500. As the physician moves the drill, computer 120 recalculates and displays the new location of graphical overlay 502. The diamond shaped end of overlay 502, labeled as area 503, represents the tip of the instrument. The physician can use image 500 and overlay 502 to visualize the position and orientation of the surgical tool relative to the patient's anatomy.

In certain situations, the physician may wish to know where the tip of the instrument would be if the instrument were projected along a line given by the instrument's current trajectory. Using the system 100 at the physician's command, computer 120 may calculate and display this projection. Area 505 in Fig. 5B is a projection of graphical overlay 502. As shown, the "look-ahead" trajectory 505 of overlay 502 is displayed in a different line style than overlay 502. Computer 120 may vary the length of look-ahead trajectory 505 as directed by the physician through a suitable computer interface device, such as a keypad, mouse, or touch pad. In this manner, computer 120 assists the physician in visualizing where the instrument would be in the patient if it were advanced a predetermined distance in the patient.

Although the "look-ahead" technique described above projected the graphical representation of the instrument into the image, there is no requirement that the instrument's graphical representation be in the space of the image for look-ahead trajectory 505 to be projected into the image. For example, the physician may be holding the instrument above the patient and outside the space of the image, so that the representation of the instrument does not appear in the image. However, it may still be desirable to project look-ahead portion 505 into the image to facilitate planning of a surgical procedure.

When surgical instrument 140 is perpendicular to the plane of the fluoroscopic image, the graphical overlay of the surgical instrument essentially collapses to a point, making it difficult to view. To alleviate this problem, computer 120 may optionally use a different graphical representation of instrument 140 when the distance in the image plane between the tip and the tail of instrument 140 becomes smaller than a fixed distance (e.g., 15 pixels).

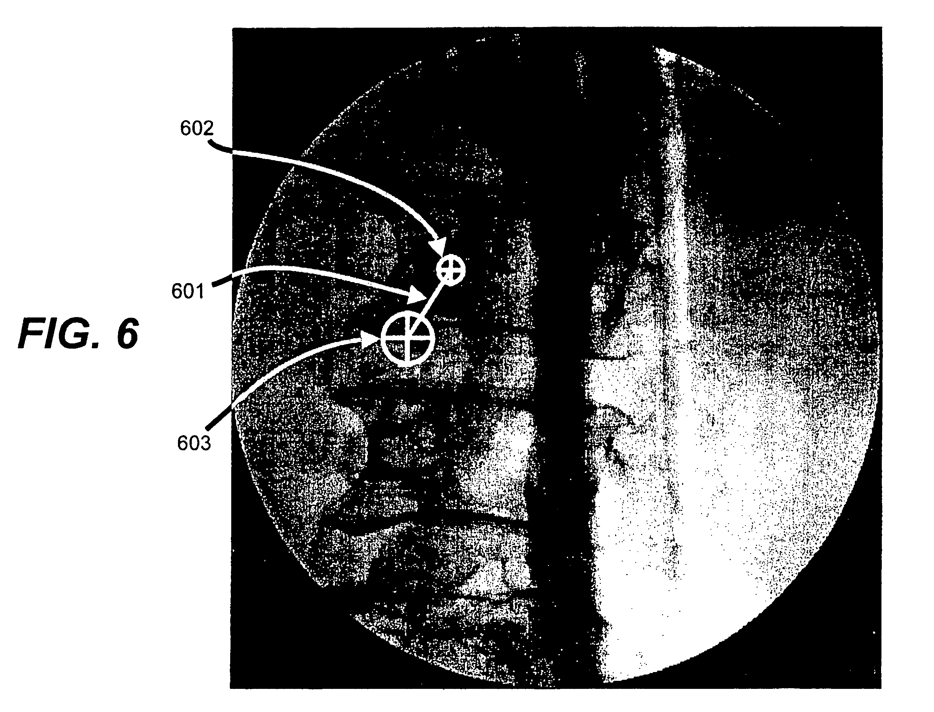

Fig. 6 is a fluoroscopic image including graphical overlay 601 of an instrument extending from a small "cross hair image," which represents tip 602 and a larger cross hair, which represents tail 603 of the instrument. Computer 120 automatically switches between the cross hair representation shown in Fig. 6 and the "straight line" representation shown in Fig. 5.

Frequently, the physician would like to acquire two complementary fluoroscopic images of the patient, such as images from an anterior/posterior view and a lateral view of the vertebral discs. The complementary views are related to one another by a rotation about an axis by a particular amount. For example, an anterior/posterior view is related to a lateral view by a 90 degree rotation around the axis running parallel through the length of the patient. When the mechanical axis of rotation of C-arm 103 is aligned with the axis relating the complementary views (e.g., when the mechanical axis is aligned with the axis running through the length of the patient), the physician can accurately and quickly switch between the complementary views by simply rotating C-arm 103 through the separation of the complementary views (usually 90 degrees). Generally, however, the axis of rotation of C-arm 103 is not inherently aligned with the axis that relates the complementary views, requiring the physician to perform a series of time consuming trial-and-error based adjustments of the fluoroscope's position through two or more axes of rotation.

In system 100 software on computer 120 allows the surgeon to easily adjust the fluoroscope's position so that one of its mechanical rotation axes such as the axis of rotation shown by arrows 108 in Fig. 1, is aligned with the axis of rotation relating the complementary views. The surgeon may then acquire the second image in the complementary image set simply by rotating C-arm 103 a certain amount, typically 90 degrees, about the aligned axis.

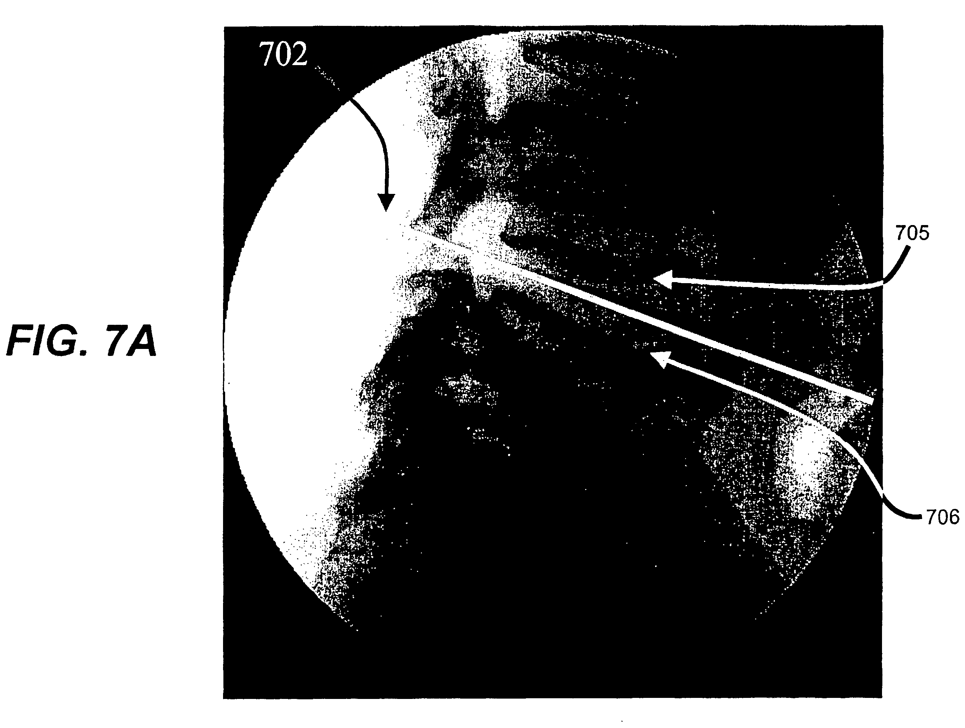

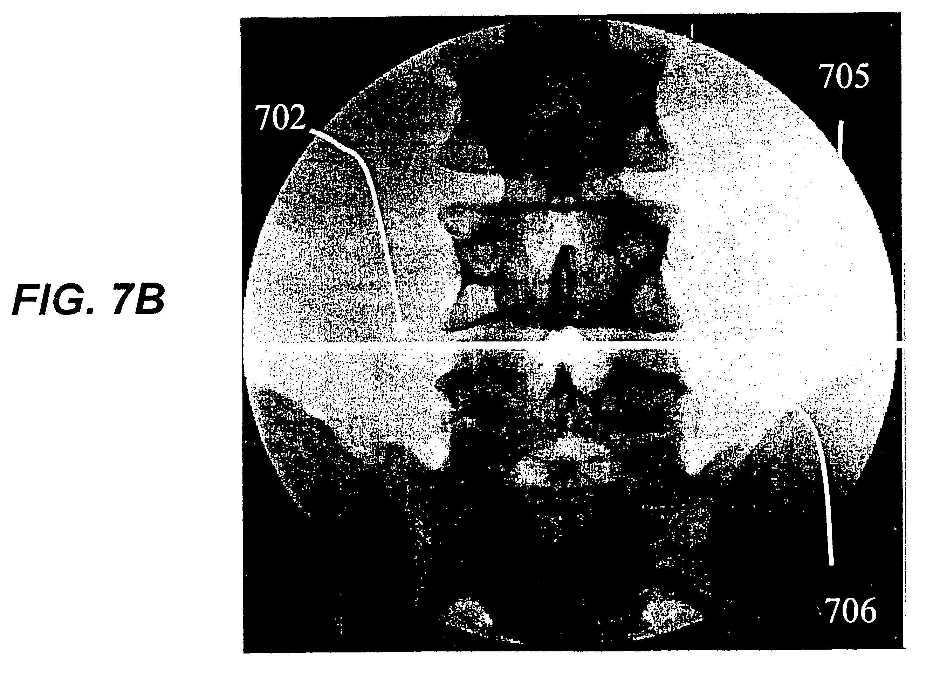

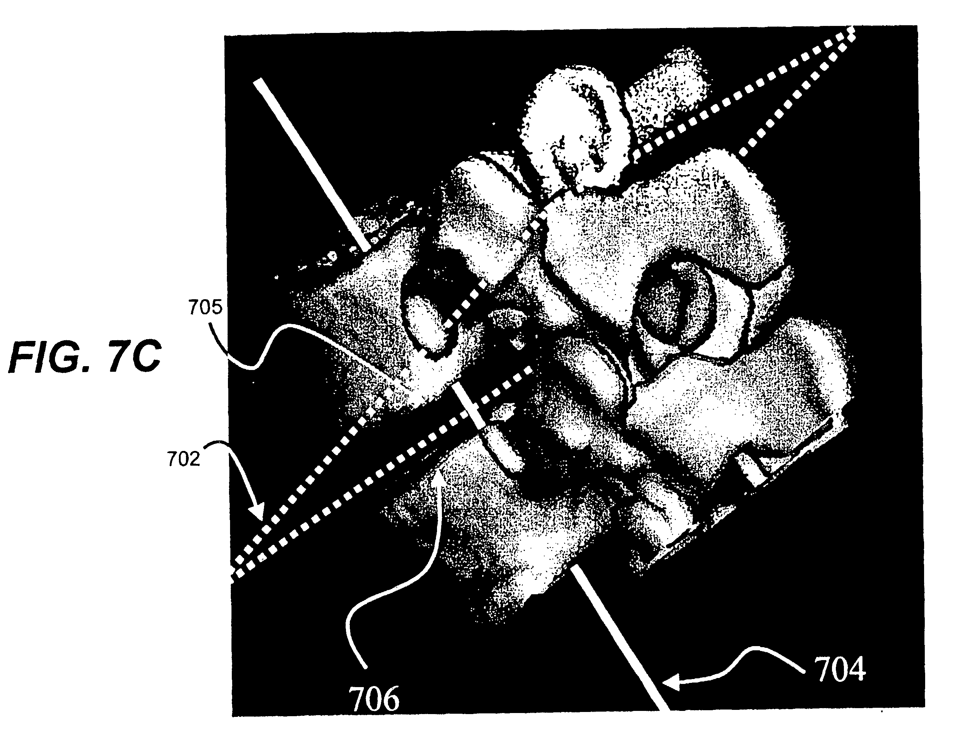

Images of complementary views and the axis that relates them are illustrated in Figs. 7A-7C. The image of Fig. 7A illustrates a lateral view of the patient's vertebral disc, in which the view direction (i.e., the direction of the central ray of fluoroscopic imager 101) is approximately parallel to the two vertebral end plates, labeled as endplate 705 and endplate 706. Line 702 is the projection of the plane substantially parallel to end plates 705 and 706. Similarly, the image shown in Fig. 7B is an anterior/posterior view of the patient's vertebral disc, in which the view direction is parallel to plane 702. The axis of rotation 704 that spatially relates the image view of Fig. 7A and the image view of Fig. 7B is a line perpendicular to plane 702. That is, rotating the image view of Fig. 7A ninety degrees about the line perpendicular to plane 702 will result in the image view shown in Fig. 7B. Fig. 7C is a three-dimensional representation of the anatomy shown in Figs. 7A and 7B. The line perpendicular to plane 702 is shown by axis of rotation 704.

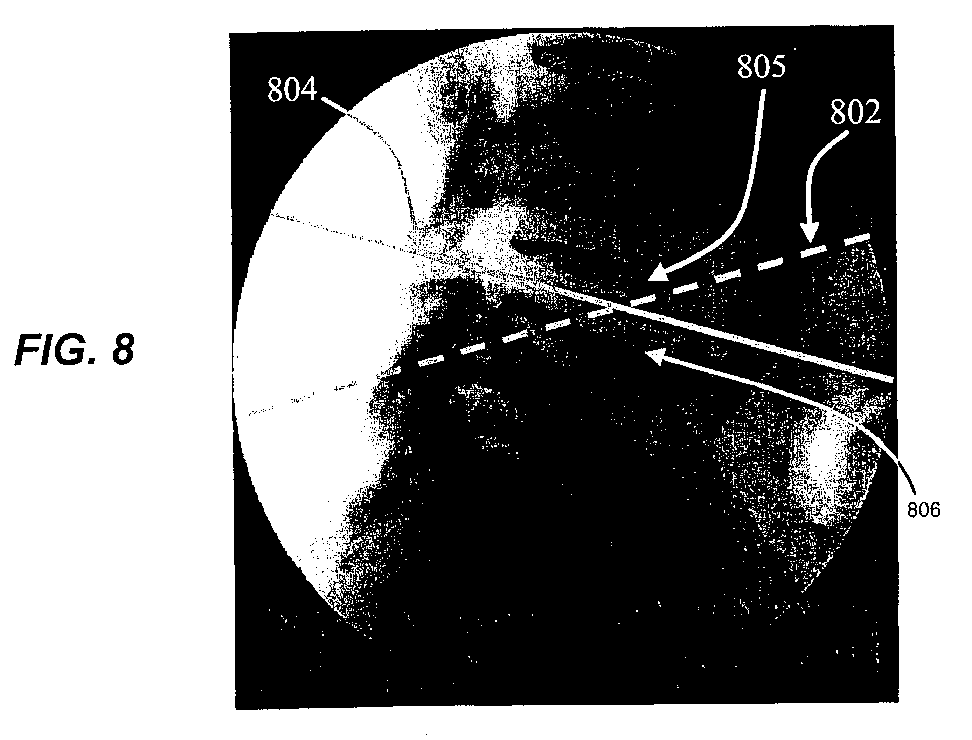

Fig. 8 is an image of a lateral view of the patient's vertebral disc, similar to Fig. 7A. In Fig. 8, however, computer 120 has drawn line 802, which represents the projection of a plane that is perpendicular to one of the C-arm's mechanical axes. Line 804 represents the plane that spatially relates the complementary views. With line 802 visible, the physician may adjust the position of fluoroscopic imager 101 so that line 802 is lined up with line 804. At this point, switching between the complementary views simply involves rotating C-arm 103 about a single mechanical axis.

Although the alignment of lines 802 and 804, as discussed above, was illustrated using both lines 802 and 804 drawn on the fluoroscopic image, in practice, it may only be necessary to display line 802 in the image. In this case, line 804 is mentally visualized by the physician. Additionally, although the relation of complimentary views was discussed using the example of the spine, complimentary fluoroscopic images of other anatomical regions, such as, for example, the pelvis, femur, or cranium, may similarly be obtained by application of the above discussed concepts.

Before, or during, surgery, the physician may find it desirable to input an operation "plan" to computer 120. The plan may, for example, specify a desired trajectory of a surgical instrument superimposed on a fluoroscopic image. During the surgical navigation process, the goal of the surgeon would be to align the graphical icon representing the real-time location of the surgical instrument with the graphical overlay representing the planned trajectory.

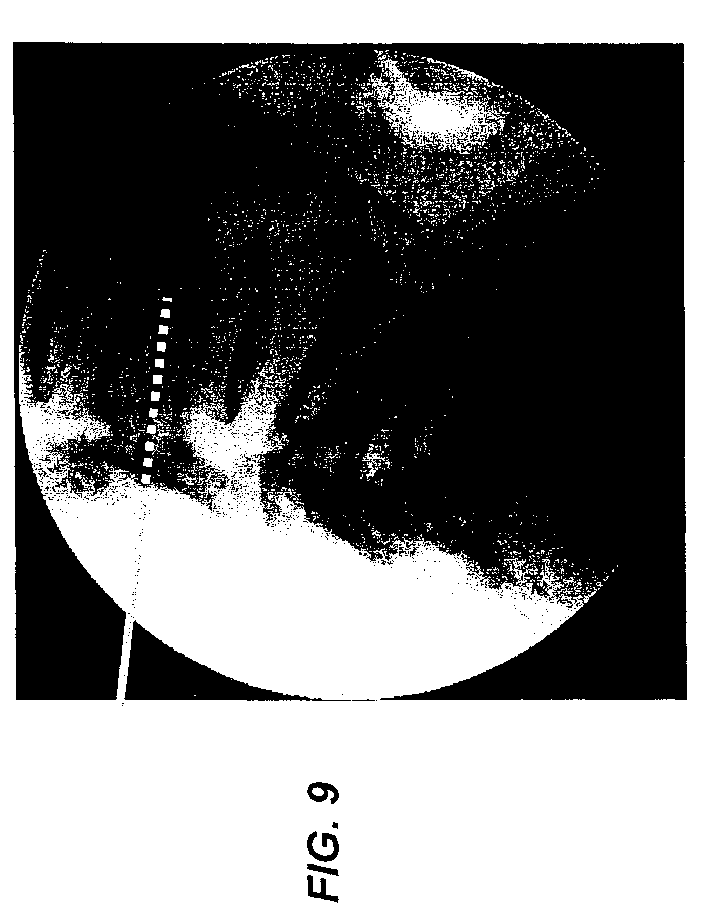

Fig. 9 is an image of a lateral view of a spinal vertebra. Assume the goal of the operation plan is to define a line that passes along a desired trajectory within the image of the vertebra. One method of accomplishing this goal is to directly input the desired trajectory information to computer 120 using traditional computer input devices. While this method of directly interacting with computer 120 is possible, it can be cumbersome and disruptive during surgery. Consistent with an aspect of the present invention, an alternative method of accomplishing this is for the physician to position the surgical instrument on the surface of the bone or skin in the desired orientation, and then project the tip of the instrument forward using the previously described look-ahead technique. More specifically, the desired trajectory is specified by (1) adjusting the position and orientation of the instrument near the patient with virtual look-ahead active, and (2) adjusting the length of the virtual look-ahead. Fig. 9 illustrates the iconic representation of instrument 901 and the virtual look-ahead projection of the instrument 902. Once the desired trajectory is achieved, the surgeon may direct computer 120 to "freeze" the planned trajectory on display 121. The desired trajectory can be obtained using one or more C-arm fluoroscopic images with two or more being required to define a specific three-dimensional trajectory which can then be displayed on any C-arm fluoroscopic view. The freeze operation may be input to computer 120 through, for example, a simple input device such as a foot pedal. The physician may then proceed with the operation, using the overlay of the planned target as a guide.

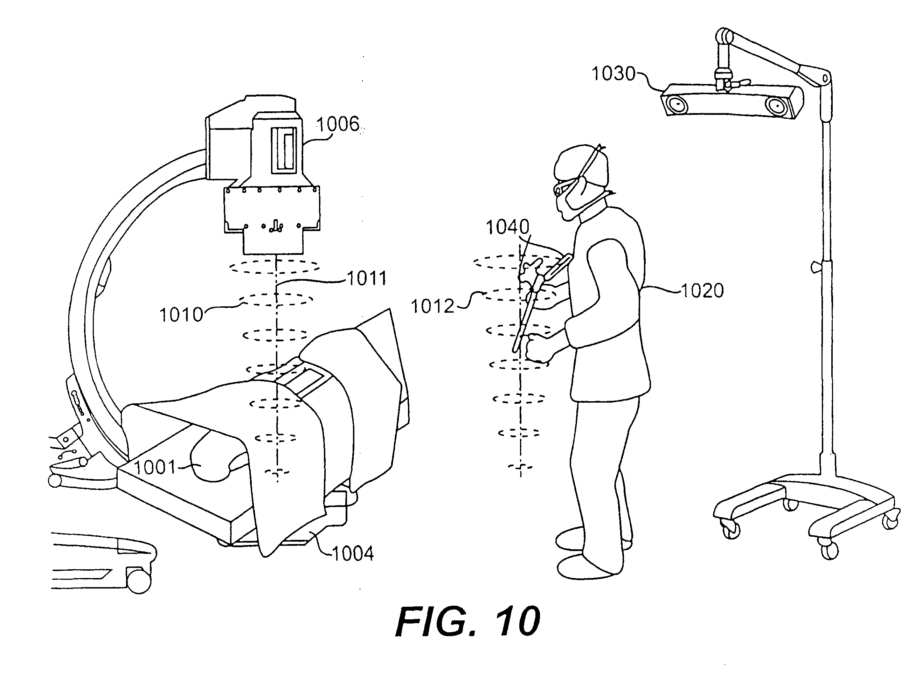

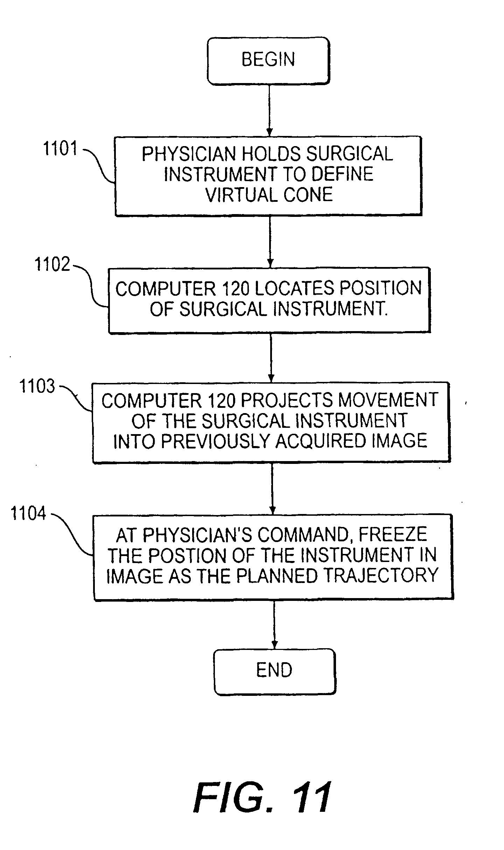

Yet another method consistent with the present invention for specifying a planned trajectory of a surgical instrument, which, unlike the method discussed above, does not require positioning the surgical instrument on or near the patient's bone, is illustrated in Figs. 10 and 11.

As shown in Fig. 10, during the acquisition of an image, patient 1001 is positioned between C-arm x-ray source 1004 and x-ray receiving section 1006. Fluoroscopic images of patient 1001 are created by the x-rays emitted from x-ray source 1004 as they travel in the path generally outlined by cone 1010 through patient 1001. Line 1011, in the center of cone 1010, corresponds to the origin (i.e., the center point) in the acquired fluoroscopic images. Physician 1020, standing within the range of tracking sensor 1030, but away from patient 1001, commands the computer to create an explicit correspondence between the fluoroscope's imaging cone 1010 and a "virtual" cone 1012 at an arbitrary position in space (which is visible to the tracking sensor). Once this virtual cone has been defined, the surgical instrument 1040 can be projected from this virtual cone into one or more pre-acquired fluoroscopic images in the same manner as if the instrument were located in the actual cone 1010 corresponding to a given image. In this manner, physician 1020 can plan the trajectory of surgical instrument 1040 by simply moving the instrument in the coordinate system established by the virtual cone.

To define the correspondence between actual and virtual cones, it is necessary for the physician to define the position of the virtual cone relative to the tracking sensor. In general, there are many ways to define a cone in space. For example, the position and orientation of a cone can be defined by three points, one corresponding to its apex, one corresponding to a second point along its central axis, and a third corresponding to the rotation of the cone about the central axis. Therefore, one way to define the cone would be to use the tip of the surgical instrument to define these three points in space relative to the tracking sensor. Another way to define this correspondence is to use a single measurement of a surgical instrument. Using this method, the axis of the instrument corresponds to the axis of the cone, the tip of the instrument corresponds to a fixed point along the axis of the cone (which could be the apex, but could also be another point along the central axis), and the orientation of the instrument about its axis corresponds to the orientation of the cone about its axis. In general any set of measurements which define the position and orientation of a given cone can be used to establish the correspondence between the actual and virtual cones.

The operations illustrated in Fig. 10 are shown in the flowchart of Fig. 11. To begin the physician holds the surgical instrument 1040 in the position that defines the virtual cone in the manner as outlined in the previous paragraph (step 1101). Computer 120 locates the position of instrument 1040, which effectively corresponds the position and orientation of the virtual cone to the actual cone (step 1102). Computer 120 projects additional movements of instrument 1040 into one or more previously acquired fluoroscopic images as if the instrument were being moved in the actual cone corresponding to a given image (step 1103). In this manner, the physician can align the instrument to particular points or trajectories within previously acquired images. At the physician's command, computer 120 "freezes" the position and/or orientation of the instrument in the displayed fluoroscopic image(s) and uses those for subsequent processing and plan generation (step 1104) it is also consistent with this invention to provide automated planning using computer analysis techniques to define an "optimal" trajectory in the C-arm images. Once the optimal trajectory is determined, computer 120 overlays the optimal trajectory in the fluoroscopic image. For example, automated plans can be generated using computational techniques to reduce a specified amount of lordosis in spine surgery.

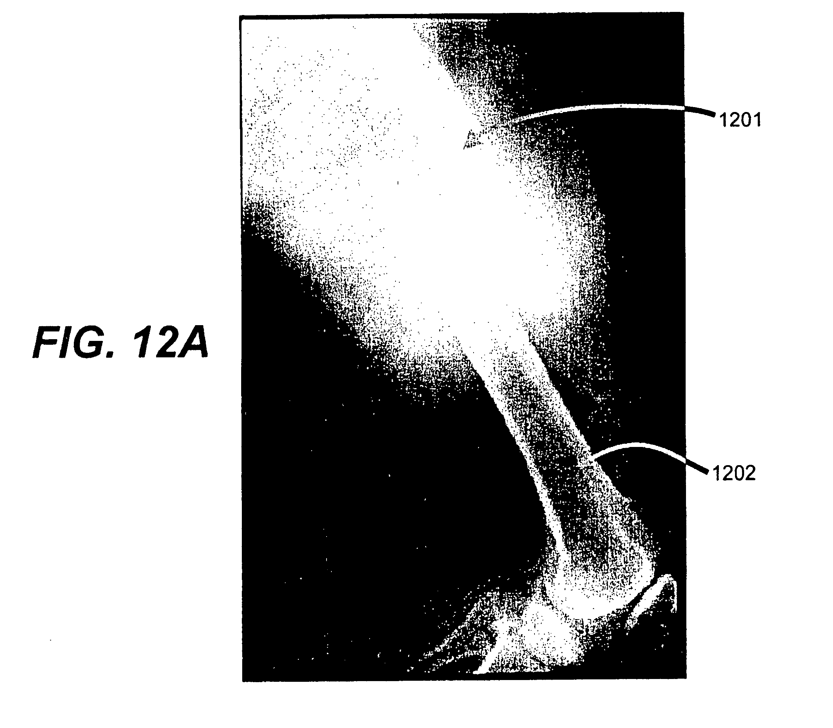

A common clinical problem, especially in orthopaedic trauma, is the realignment (reduction) of broken or misaligned bone fragments. Fig. 12A is a fluoroscopic image of a fracture of the femur containing two bone fragments 1201 and 1202. The physician's job is to realign the bone fragments so that the femur can properly heal.

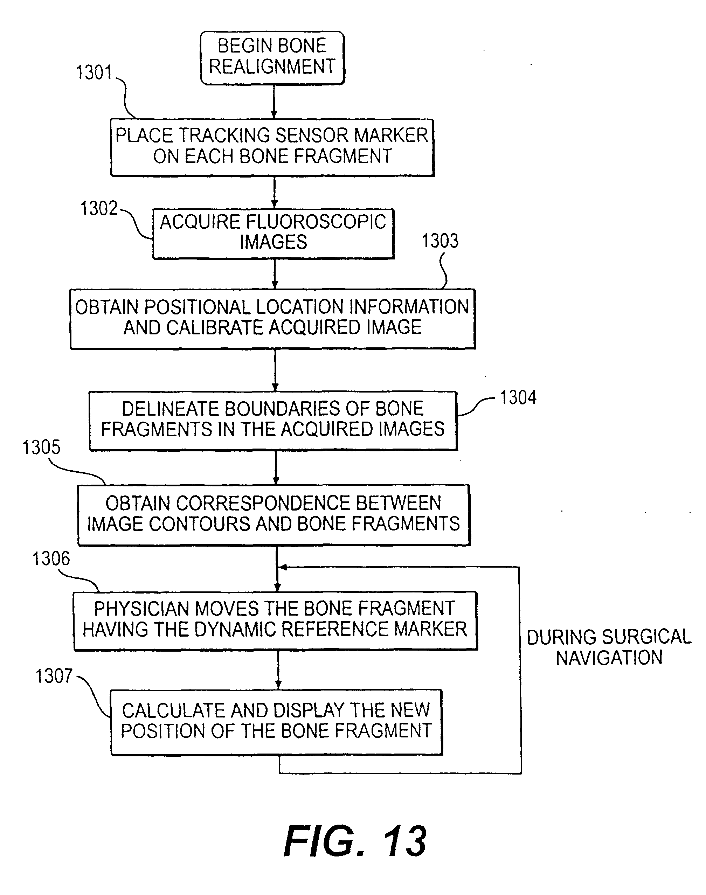

Fig. 13 is a flow chart illustrating methods for aligning bone fragments. In general, one of bone fragments 1201 or 1202 is used as a fixed reference frame and the other as a dynamic reference frame. When the physician moves the bone fragment corresponding to the dynamic reference frame, tracking sensor 130 detects the movement and updates the x-ray image to reflect the new location of the bone fragment in the patient.

To begin the alignment procedure, the physician places a tracking sensor marker on each of bone fragments 1201 and 1202 (step 1301) and acquires the fluoroscopic images, (step 1302), such as the image shown in Fig. 12A. Computer 120 processes the acquired image to obtain positional location information and to calibrate the image (step 1303, this step is identical to steps 401-403 in Fig. 4).

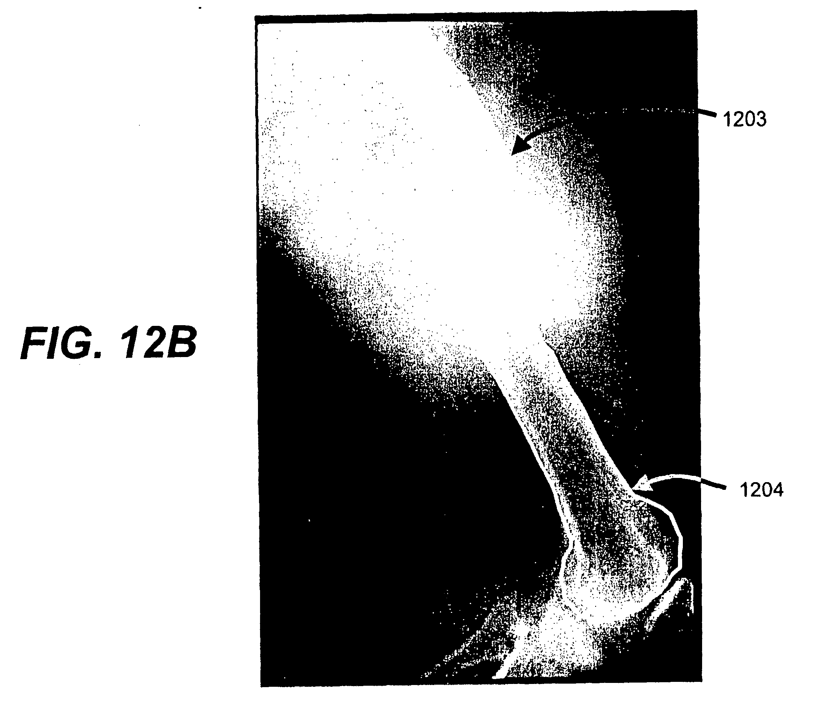

After acquisition of the fluoroscopic image(s), computer 120 uses image detection and extraction techniques to delineate the boundaries of the bone fragments in the images (step 1304). Suitable edge detection algorithms for generating the contours are well known in the art, and may be, for example, the Canny edge detector, the Shen-Casten edge detector, or the Sobel edge detector. An edge detected version of Fig. 12A is shown in Fig. 12B, in which the resulting contour corresponding to bone fragment 1201 is labeled as 1203 and the contour corresponding to bone fragment 1202 is labeled as 1204. Contours 1203 and 1204 may be, as shown in Fig. 12B, graphically superimposed by computer 120 on the acquired image(s).

Overlaying the detected image contours on the fluoroscopic image allows the physician to easily identify the correspondence between image contours 1203 -1204 and bone fragments 1201-1202. The physician inputs this correspondence into computer 120 (step 1305). Alternatively, computer 120 may automatically identify the correspondence between the image contours and the bone fragments. Once the correspondence is established, the physician specifies which contour is to remain fixed and which is to be repositioned. The tracking sensor marker attached to the fragment to be repositioned is referred to as the dynamic reference marker and the tracking sensor marker attached to the fixed fragment is referred to as the fixed reference frame marker, although physically the dynamic reference marker and the fixed reference frame marker may be identical.

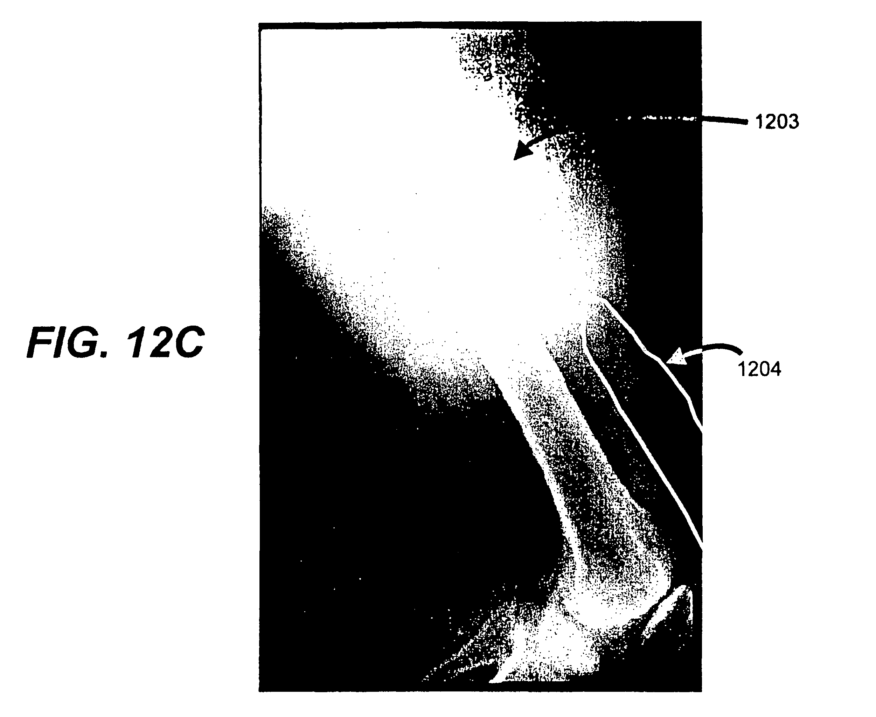

During surgical navigation, the physician moves the bone fragment having the dynamic reference marker (step 1306). Tracking sensor 130 detects the position of the dynamic reference frame marker and the fixed frame marker. With this information and the previously generated positional location information, computer 120 calculates and displays the new position of the dynamic reference frame, and hence its corresponding bone fragment, in the fluoroscopic image (step 1307). Fig. 12C illustrates an updated version of the fluoroscopic image contour 1203 corresponding to the fixed bone fragment and contour 1204 corresponding to the new location of the dynamic reference marker and its bone fragment.

Methods described above for aligning bone fragments may also be applied to the proper alignment of multiple vertebral bodies, for example in the reduction of scoliosis.

The navigational guidance system 100 used by the present invention is not limited to providing surgical navigational guidance with two-dimensional fluoroscopic images. Three-dimensional volumetric data sets may also be overlaid with graphical representations of a surgical instrument. Three-dimensional data sets (such as CT or MRI) may be either pre-acquired or acquired during the operation.

Two types of three-dimensional data sets are typically used in surgical navigation: patient-specific image data and non-patient specific or atlas data. Patient-specific three-dimensional images are typically acquired prior to surgery using computed tomography (CT), magnetic resonance (MR), or other known three-dimensional imaging modalities, although intra-operative acquisition is also possible. Atlas data is non-patient specific three-dimensional data describing a "generic" patient. Atlas data may be acquired using CT, MR or other imaging modalities from a particular patient; and may even comprise images from several modalities which are spatially registered (e.g., CT and MR together in a common coordinate system). Atlas data may be annotated with supplemental information describing anatomy, physiology, pathology, or "optimal" planning information (for example screw placements, lordosis angles, scoliotic correction plans, etc).

A three-dimensional patient CT or MR data set is shown in Fig. 1 as data set 124 and atlas data is illustrated in Fig. 1 as data set 126.Before overlaying a three-dimensional image with graphical representations of surgical instruments, the correspondence between points in the three-dimensional image and points in the patient's reference frame must be determined. This procedure is known as registration of the image. One method for performing image registration is described in the previously mentioned publications to Bucholz. Three-dimensional patient specific images can be registered to a patient on the operating room table (surgical space) using multiple two-dimensional image projections. This process, which is often referred to as 2D/3D registration, uses two spatial transformations that can be established. The first transformation is between the acquired fluoroscopic images and the three-dimensional image data set (e.g., CT or MR) corresponding to the same patient. The second transformation is between the coordinate system of the fluoroscopic images and an externally measurable reference system attached to the fluoroscopic imager. Once these transformations have been established, it is possible to directly relate surgical space to three-dimensional image space.

When performing three-dimensional registration, as with two-dimensional registration, imager 101, when acquiring the image, should be stationary with respect to patient 110. If C -arm 103 or patient 110 is moving during image acquisition, the position of the fluoroscope will not be accurately determined relative to the patient's reference frame. Accordingly, the previously described technique for detecting movement of imager 101 during the image acquisition process can be used when acquiring fluoroscopic images that are to be used in 2D/3D registration. That is, as described, computer 120 may examine the position information from tracking sensor 130 while radiation sensors 107 are signaling radiation detection. If the calibration and tracking target 106 moves relative to dynamic reference frame 150 during image acquisition, this image is marked as erroneous.

It may be necessary to acquire complementary fluoroscopic views (e.g., lateral and anterior/posterior) to facilitate 2D/3D registration. The techniques previously discussed in reference to Figs. 7-8 and relating to the acquisition of complementary views can be applied here.

Once registered, computer 120 may use positional information of instrument 140 to overlay graphical representations of the instrument in the three-dimensional image as well as the two-dimensional fluoroscopic images.

### Hybrid Use of Three-Dimensional and Two-Dimensional Image Data

The two-dimensional images generated by imager 101 are not always able to adequately represent the patient's bone structure. For example, fluoroscopic x-ray images are not effective when taken through the length of the patient (i.e., from the point of view looking down at the patient's head or up from the patient's feet) because the large number of bones that the x-rays pass through occlude one another in the final image. However, information required for planning a surgical procedure which is not otherwise available based on two-dimensional image data alone may be extracted from a three-dimensional image data set such as a CT or MR image data set. The extracted information may then be transferred to the two-dimensional x-ray images generated by imager 101 and used in surgical navigation. The following examples describe additional methods for using three-dimensional and two-dimensional data in surgical navigation.

### Example 1: Importing Three-Dimensional Surgical Implant Specifications to Two-Dimensional Images

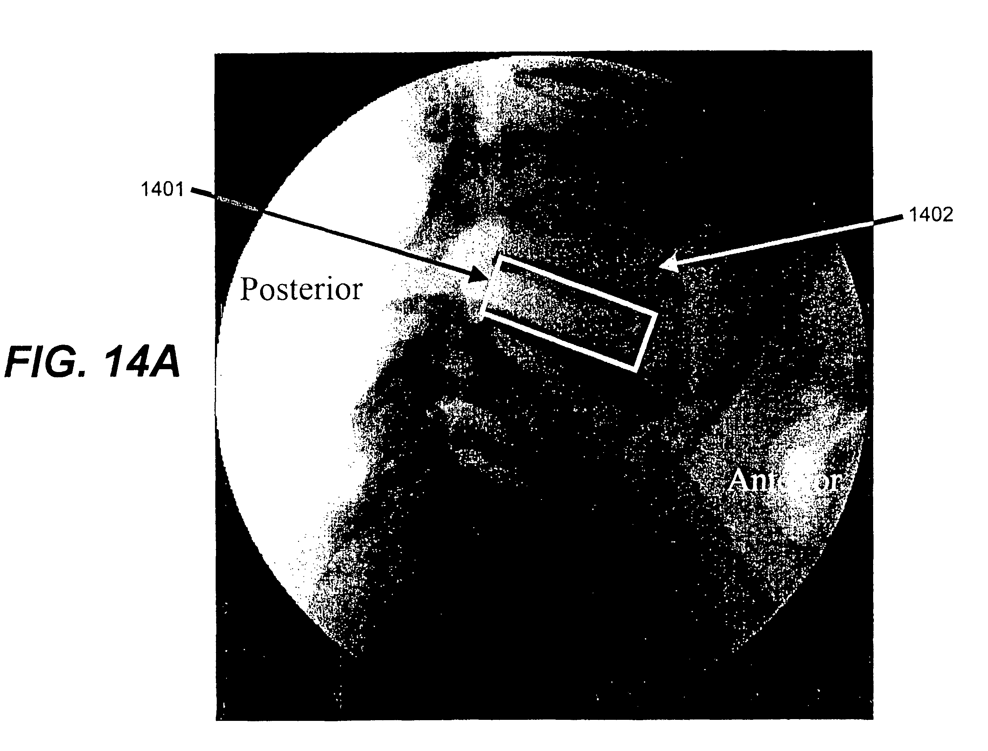

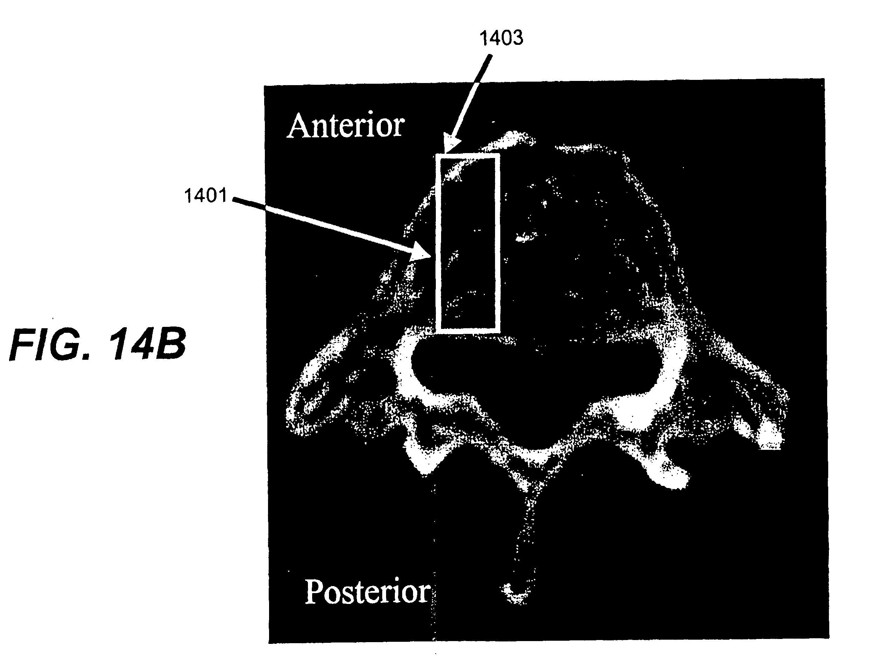

Figs. 14A and 14B are images illustrating the implantation of an inter-vertebral cage in the spine of a patient using the system 100 (but not using the present invention). An inter-vertebral cage is a roughly cylindrical spinal implant that is inserted in the disc space between adjacent spinal vertebrae. The physician may find it difficult, if not impossible, to choose the appropriate length of an inter-vertebral cage based upon two-dimensional images such as the image of Fig. 14A.

Rectangle 1401 represents the projection of the cylindrical inter-vertebral cage into the image. While the long axis of the cylinder appears to be completely within the bone in this image, this may not be the case due to curvature of the anterior aspect of vertebrae 1402. Fig. 14B is an image of a three-dimensional axial CT cross section of the vertebrae. Comer 1403 of rectangle 1401 protrudes from the bone-a highly undesirable situation that cannot be reliably detected in x-ray images such as that of Fig. 14A. Accordingly, when faced with this situation, the appropriate cage length should be chosen based upon one or more axial CT images, such as that in Fig. 14B. Selection of the cage length can be performed automatically by computer 120 or semi-automatically with the input of the physician.

Once the cage length has been determined by the physician and entered into computer 120, the length value can then be used by computer 120 in properly displaying the graphical overlay in the associated two-dimensional image. The position of the surgical instrument used to hold the cage during the insertion process, as detected by tracking sensor 130, is used to calculate the position of the cage in Fig. 14A during the two-dimensional navigational process.

Although the above discussed example was with a cylindrical spinal implant, in general, the described concepts could be applied to any surgical implant.

### Example 2: Acquisition of an X-Ray View Down the Medial Axis of a Vertebral Pedicle

In certain clinical procedures, it may be desirable to acquire a fluoroscopic x-ray image view looking substantially straight down the medial axis of a vertebral pedicle. For the purposes of this example, a vertebral pedicle can be thought of as a cylinder, and the medial axis corresponds to the central axis of the cylinder.

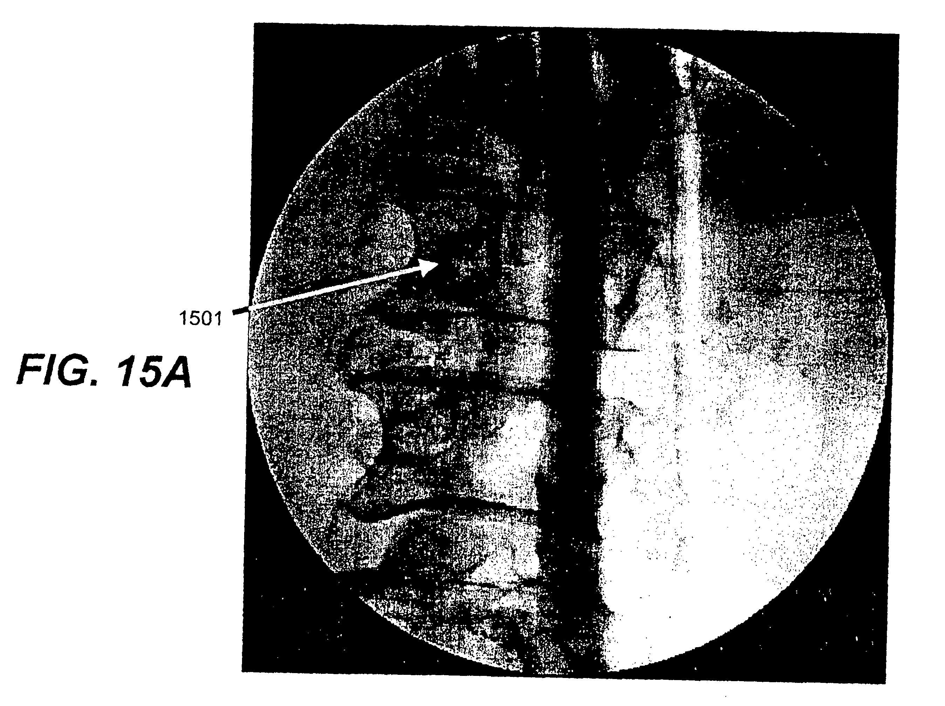

Fig. 15A is an x-ray image in which the view direction of the imager is aligned with the medial axis of the pedicle (i.e., the medial axis of the pedicle is into the plane of the image). In this so-called "owl's eye" view, the pedicle appears as circle 1501 within the image. It is often difficult to precisely acquire this view using only fluoroscopic x-ray images, as it is difficult to align the view direction of imager 101 with the medial axis of the pedicle using only fluoroscopic images.

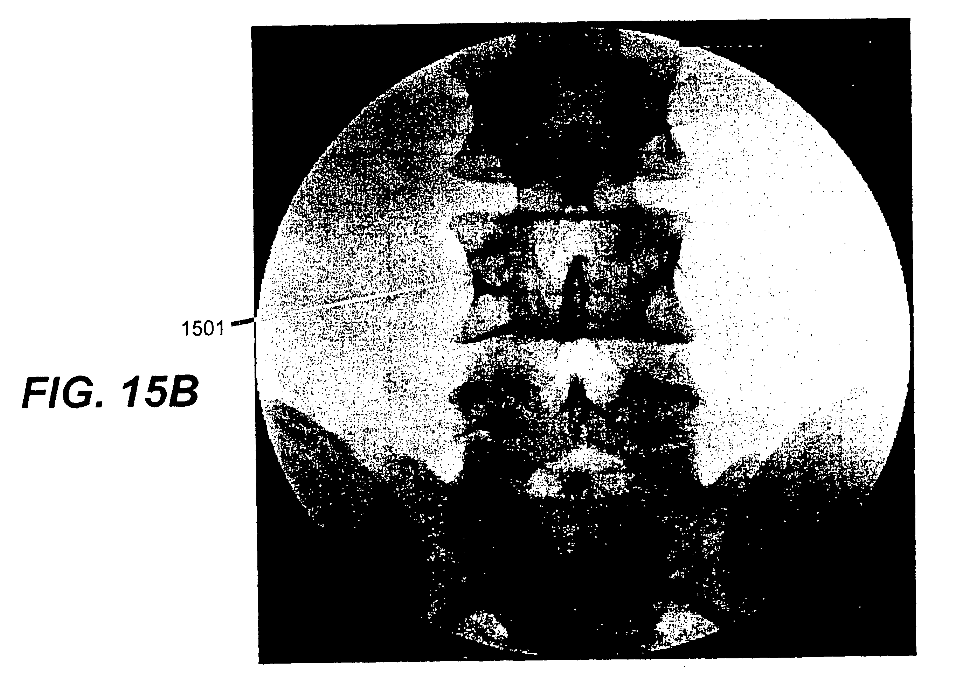

Given an anterior/posterior fluoroscopic image view of the spine, such as the one shown in Fig. 15B, and given that the mechanical axis of the fluoroscope is aligned with the patient's long axis (i.e., axis 704 in Fig. 7C), an axial CT cross section of a vertebra can be used to quickly and easily acquire a high quality owl's eye view, such as the view of Fig. 15A.

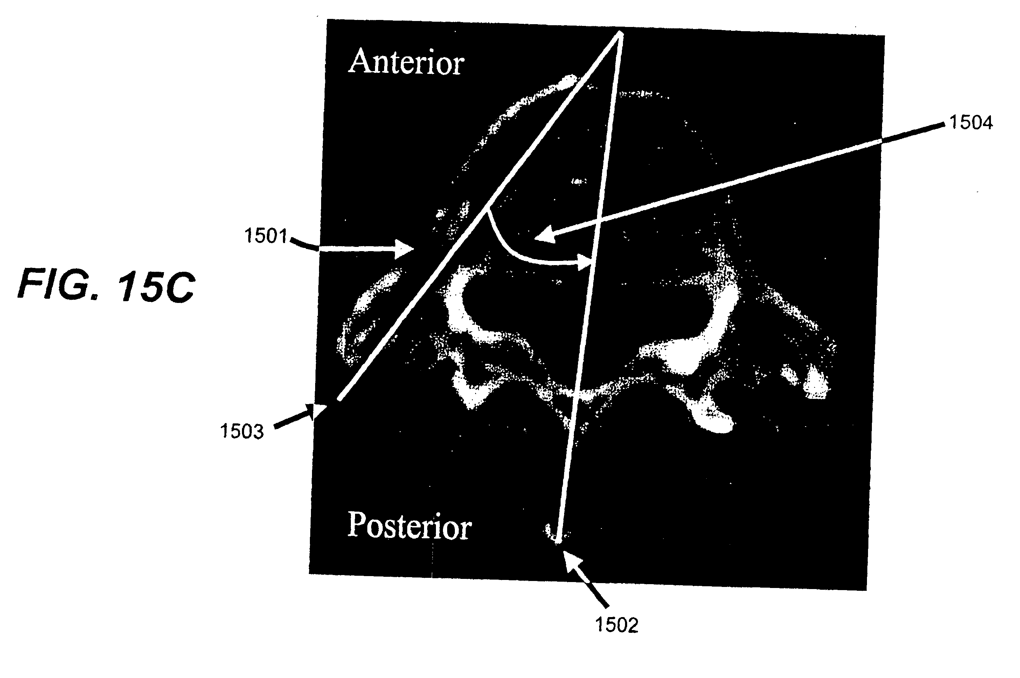

Fig. 15C is an image of an axial CT cross section of a vertebra. With this image, computer 120 or the physician may measure angle 1504 between the anterior/posterior axis 1502 and the projection of the medial axis 1503 of the pedicle 1501 into the axial plane. The physician may then rotate imager 101 by the measured angle about the mechanical rotation axis that is aligned with the patient's long axis 704. Because most fluoroscopic imagers, such as imager 101, have angle indicators, rotation by the desired amount is trivial. However, if the physician requires additional accuracy in the rotation, tracking sensor 130, because it detects the position of C-arm 103, can be used to more precisely measure the rotation angle.

### Example 3: Use of Digitally Reconstructed Radiography in the Placement of a Surgical Implant

With conventional fluoroscopic x-ray image acquisition, radiation passes through a physical media to create a projection image on a radiation sensitive film or an electronic image intensifier. Given a 3D CT data set, a simulated x-ray image can also be generated using a technique known as digitally reconstructed radiography (DRR). DRR is well known in the art, and is described, for example, by L. Lemieux et al., "A Patient-to-Computed-Tomography Image Registration Method Based on Digitally Reconstructed Radiographs," Medical Physics 2 1 (11), pp 1749-1760, November 1994.

When a DRR image is created, a fluoroscopic image is formed by computationally projecting volume elements (voxels) of the 3D CT data set onto a selected image plane. Using a 3D CT data set of a given patient, it is possible to create a DRR image that appears very similar to a corresponding x-ray image of the same patient. A requirement for this similarity is that the "computational x-ray imager" and actual x-ray imager use similar intrinsic imaging parameters (e.g., projection transformations, distortion correction) and extrinsic imaging parameters (e.g., view direction). The intrinsic imaging parameters can be derived from the calibration process.

A DRR image may be used to provide guidance to the surgeon in the problem discussed in Example 1 of appropriately placing an inter-vertebral cage in the patient. Given a 3D CT data set of two adjacent vertebrae, the physician, interacting with computer 120, may manually position a 3D CAD model of an inter-vertebral cage in a clinically desired position in the three-dimensional view of the vertebrae. The physician may then use the DRR technique to synthesize an anterior/posterior, lateral, or other x-ray view of the vertebrae showing the three-dimensional CAD model of the inter-vertebral cage. Thus, a synthetic fluoroscopic x-ray image can be created which simulates what a property placed cage would look like after implantation.

The simulated x-ray images may be compared to the actual images taken by imager 101 during surgery. The goal of the surgeon is to position the implant such that the intra-operative images match the DRR images. For this comparison, two types of intra-operative images may preferably be used. First, conventional fluoroscopy could be used to acquire an image after the inter-vertebral cage has been implanted. Second, images acquired prior to cage placement could be supplemented with superimposed graphical icons representing the measured cage position. In either case, the synthetic fluoroscopic image can be used as a template to help guide the surgeon in property placing the inter-vertebral cage.

Although the above example was described in the context of implanting an intervertebral cage, implants other than the inter-vertebral cage could also be used.

### Example 4: Obtaining a Particular Two-Dimensional View Direction Using Digitally Reconstructed Radiograph Images

The DRR technique can be used to provide guidance to the physician when acquiring an owl's eye view of a vertebral pedicle. Given a three-dimensional CT data set containing a vertebra and associated pedicle, the physician may use computer 120 to manually locate a three-dimensional representation of the pedicle's medial axis relative to the three-dimensional images of the vertebrae. Once this placement has been achieved, it is possible to synthesize an owl's eye view of the vertebrae based upon the view direction specified by the physician's selection of the three-dimensional medial axis. This synthetic image can then be displayed to the surgeon during surgery and used to guide the acquisition of an actual owl's eye view using the fluoroscope. By visually comparing fluoroscopic images taken while positioning the fluoroscope to the synthetic owl's eye view, the physician can acquire a fluoroscopic image with a view direction approximately equal to the manually selected medial axis. In this manner, a high quality owl's eye view can be acquired.

Although the above example was described in the context of synthesizing a two-dimensional owl's eye view, in general, any three-dimensional view direction can be selected and a corresponding two-dimensional image synthesized and used to acquire a fluoroscopic two-dimensional image.

### Example 5: Measuring Out-Of-Plane Angles Based On Fluoroscopic Images

It may be desirable to measure the angle between the trajectory of a surgical instrument and the plane of a fluoroscopic image (such as a plane aligned with the mid-line of the spine 1502) during surgery using a pre-acquired fluoroscopic image. This is useful, as it is often desirable to position or implant a surgical instrument at a certain angle relative to the plane of the fluoroscopic image. For example, the surgical instrument may need to be implanted in the direction aligned with the medial axis of the pedicle 1503.

Consider the vertebral cross section shown as an axial CT image in Fig. 15C. As described above, the angle 1504 between the anterior/posterior axis of the spine 1502 and the medial axis 1503 of the pedicle can be measured from this CT image. Aligning the surgical instrument with the medial axis can be accomplished by dynamically measuring the angle between the trajectory of the surgical instrument and the plane defined by the mid-line of the spine 1502. When the dynamically measured angle matches the angle pre-obtained from the CT image, the surgical instrument is aligned.

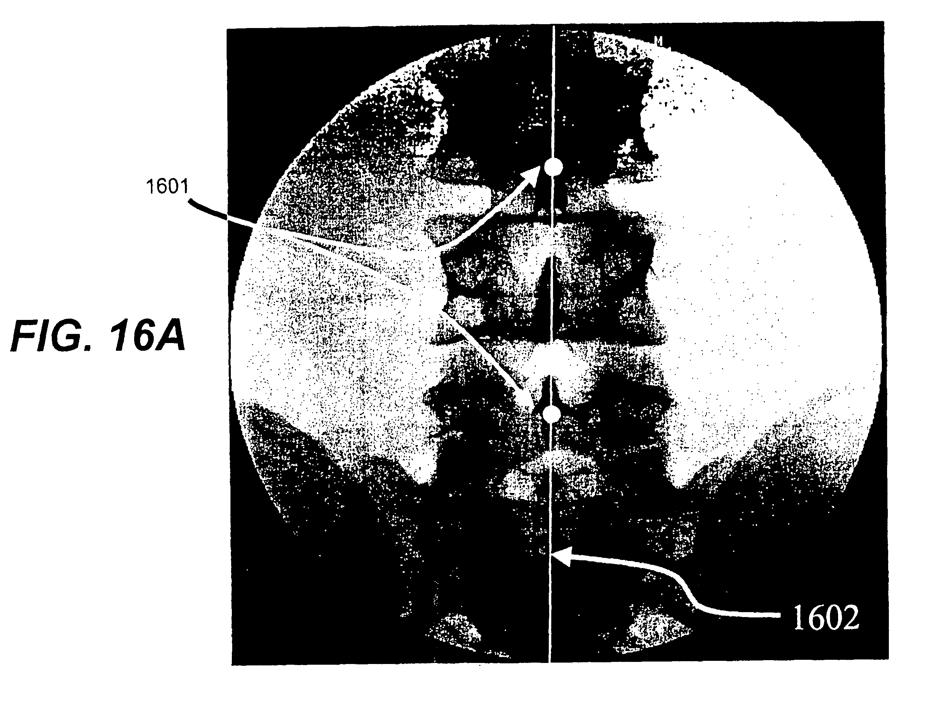

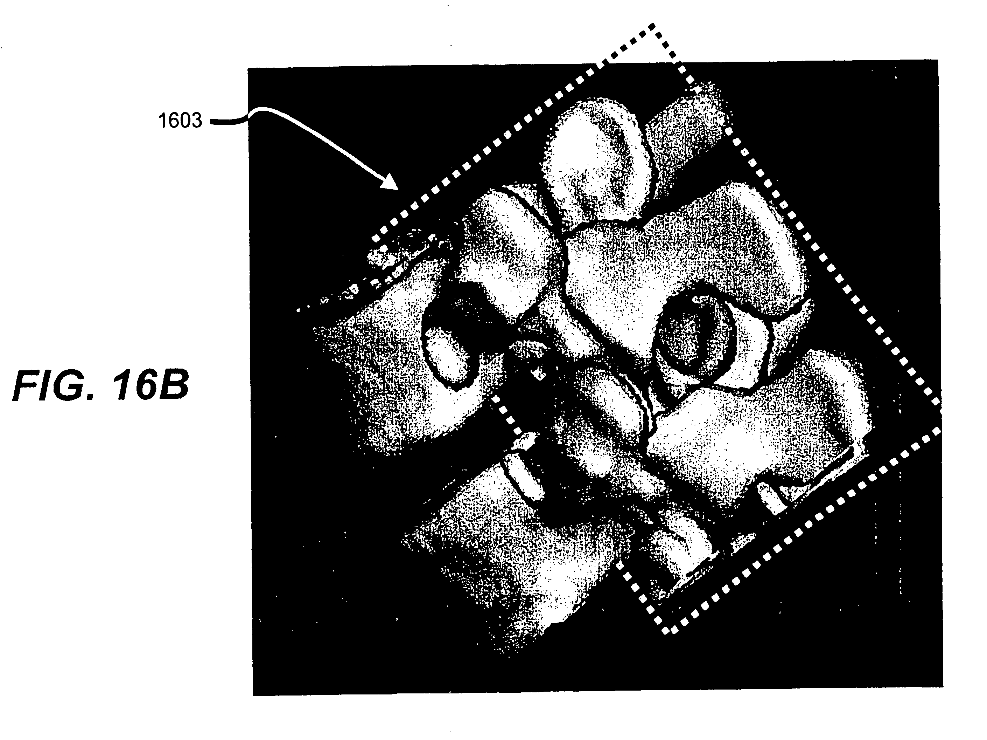

Figs. 16A and 16B are figures respectively illustrating an anterior/posterior fluoroscopic image of the spine and a corresponding three-dimensional view of the spine. In a manner to be discussed in more detail below, the physician defines two points along the midline of the spine, such as the points 1601 drawn on the spinous processes in Fig. 16A (in non-pathological anatomy a spinous process typically defines the midline). Computer 120 of system 100 uses these points to define a line 1602 in the image, or more precisely, the computer defines plane 1603 (shown in Fig. 16B) to include the two points and the linear projections of these two points dictated by the calibration transformation. More intuitively, a first order approximation of plane 1603 can be thought of as the plane passing through the two points perpendicular to the image plane.

Plane 1603 defines the midline of the spine in three-dimensional space. During navigational guidance, the equation of this plane can be expressed in the coordinate system of either the dynamic reference frame 150 or the tracking sensor 130.

Using the tracking sensor 130 to measure the position and orientation (i.e., the trajectory) of the instrument 140, computer 120 then mathematically projects this trajectory onto the plane 1603. This projection will define a line passing through plane 1603. The angle between this line in plane 1603 and the instrument trajectory corresponds to the angle to be measured. In other words, the angle to be measured corresponds to the minimum angle present between the trajectory of the instrument and the plane 1603. The angle to be measured can be calculated by computer 120 and displayed to the physician either in a textual or graphical format.

In summary, as described in this example, a single fluoroscopic image can be used during surgery to position a surgical instrument at a desired trajectory relative to the plane of the fluoroscopic image. More generally, the methods described in this example relate to measuring the angle between the trajectory of a surgical instrument 140 and a plane (e.g. 1603) defined by two or more points (e.g., 1601) which have been manually or automatically selected in a fluoroscopic image. While the explanation uses a CT for clarity of the example, the measurement and display of the angle can be achieved without the use of any 3D image data.

Although the above five examples used three-dimensional patient specific data and not atlas data, in certain situations, it may be possible to use a 2D/3D registration scheme that registers non-patient specific atlas data to patient specific fluoroscopic images using *deformable* registration methods that do not preserve the rigidity of anatomical structure during the registration process. In this manner, the patient specific fluoroscopic images may be used to deform the atlas data to better correspond to the patient and thereby transfer atlased knowledge to the patient specific fluoroscopic images.

### Anatomical Landmark Generation and Display

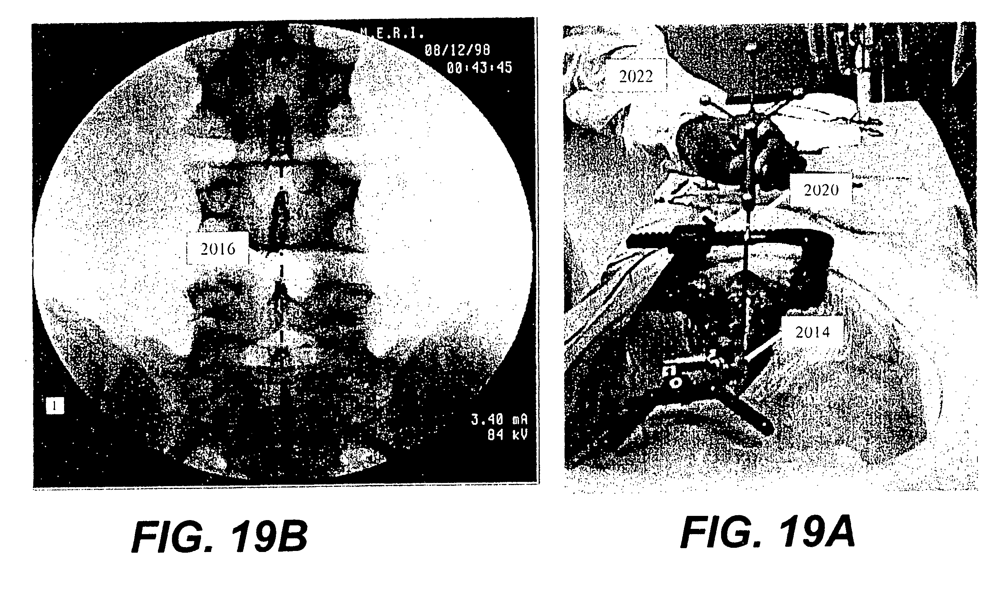

In accordance with the present invention, the apparatus includes an interface with the system computer processor for inputting one or more landmark positions to be used by the processor for generating and displaying the landmark. As embodied herein, and referring again to Fig. 1, the interface can include standard, separately provided input devices such as device 2010 operatively connected to computer 120. Input device 2010 can comprise, for example, a keyboard, a mouse, or a light pen. If the image guided system is already equipped with an input device, then the required interface can comprise suitable input instructions stored in the memory 2018 associated with computer 120 or in a separately provided, operatively connected memory device such as memory device 2026 shown in Fig. 1. Memory device 2026 can be a tape, disk, or separate computer with associated memory. Alternatively, or additionally, the required interface can include a trackable instrument such as touch-probe 2020 depicted schematically in Fig. 1 and shown in Fig. 19A. Probe 2020 includes LED array 2022 for tracking by sensor 130. By the surgeon physically touching the desired landmark positions on the patient's anatomy, apparatus 2000 can automatically determine the landmark positions in the desired coordinate reference frame through the data provided by sensor 130. Because image guided system 100 can be configured to locate a specific point on probe 2020 (i.e., the tip 2024), the requisite accuracy can be achieved.

Further in accordance with the present invention, the apparatus also includes additional computer instructions stored in a storage device accessible by the image guided surgical system computer that, when executed, cause the computer processor to generate and display the anatomical landmark positions on the display device in registration with a displayed anatomical image, that is, in the same coordinate reference frame as the image. As embodied herein, and with reference again to Fig. 1, apparatus 2000 includes appropriate additional computer instructions stored either in system memory 2018 or in separate storage device 2026.

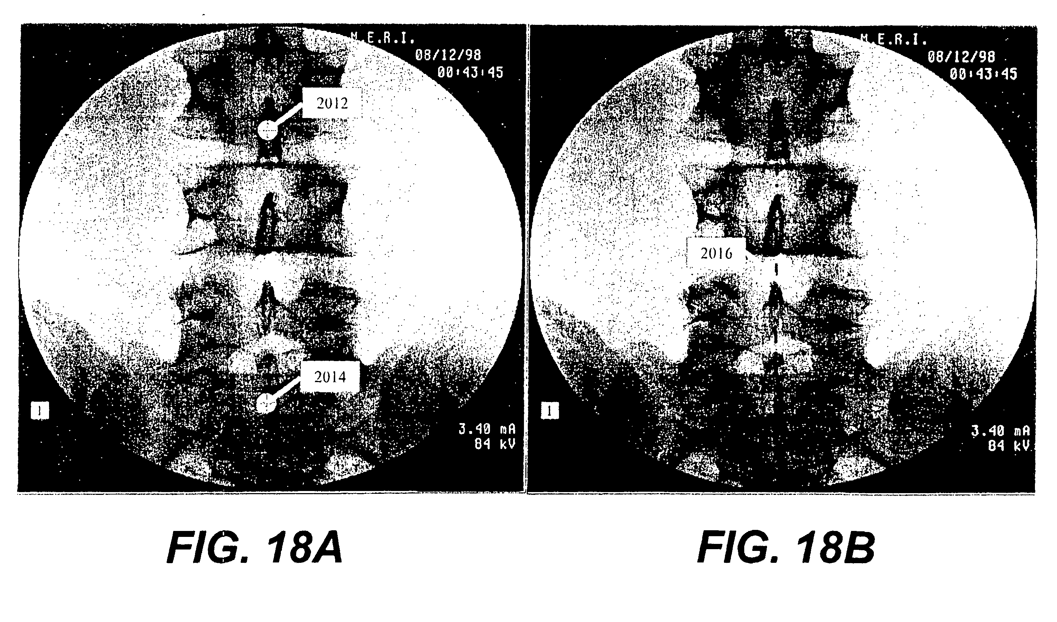

As discussed above, the additional computer instructions stored in computer memory 2018 (or in storage device 2026) cause the computer 120 to generate and display the desired landmark, namely the midline "plane" 2016 in Figs. 18B and 19B. The instructions also preferably include instructions for displaying icons or other representations of the actual inputted positions used to generate the displayed landmark, such as the iconic positions 2012 and 2014 shown in Fig. 18A. Also, the additional instructions can include instructions to store the inputted landmark positions, for example, in system memory 2018 or storage device 2026, for future reference or record keeping.

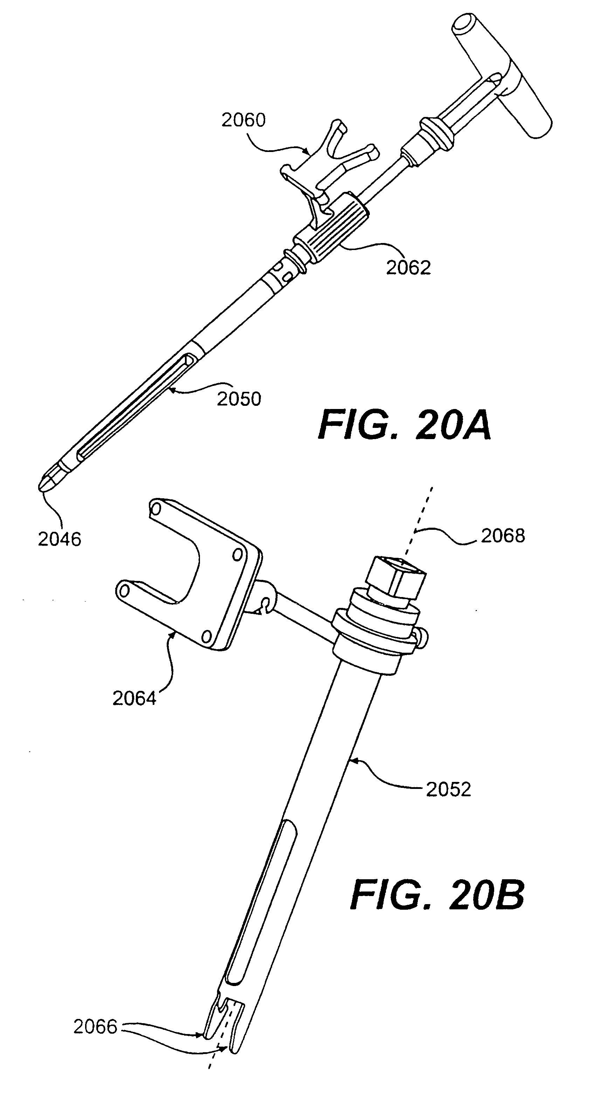

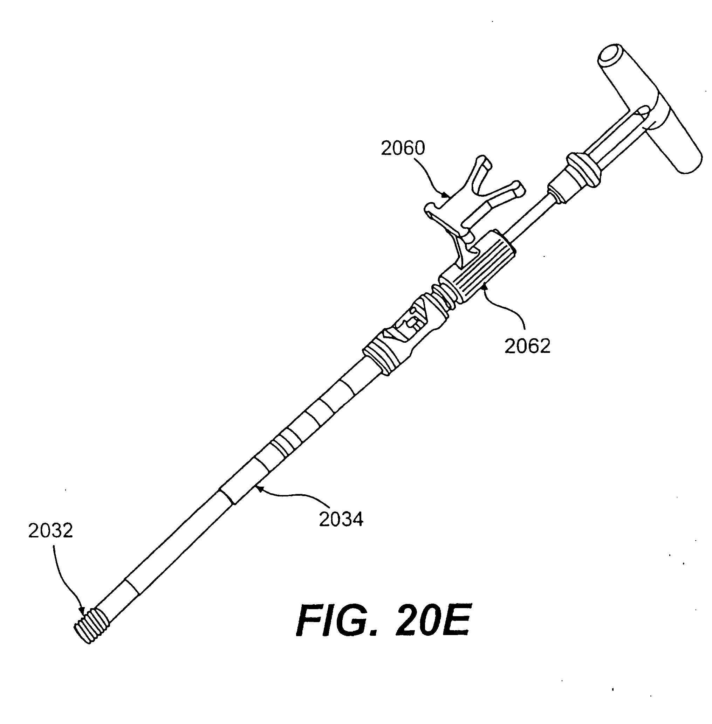

As discussed previously, the image guided surgery system 100 has the advantage of being able to track the various instruments (suitably equipped with LED emitter arrays) that may be required for a surgical procedure, and to generate and display on monitor 121, icons representing the instruments or parts thereof, e.g., the tip and tail portions, in registration with a displayed anatomical image. Thus, for an interbody fusion device implantation procedure, trackable instruments such as those shown in Figs. 20A-20E can be used with system 100 to implant an interbody fusion device, such as fusion device 2032 shown mounted on implant inserter 2034 (Fig. 20E), in a manner to be discussed in more detail henceforth.

Correct placement of the device 2032 requires the surgeon to locate the instruments relative to the midline plane 2016 so that a suitable cavity can be formed in the vertebral column a predetermined offset amount to the right or left. This offset distance is often specified by the manufacturer based on size, e.g., diameter, of device 2032 but may be selected by the surgeon. Apparatus 2000 also allows the surgeon to input via an interface such as input device 2010 the desired offset or accept a default to manufacturer's offset values which can be stored in system memory 2018 (or storage device 2026) together with appropriate instructions to calculate the offset position in the coordinate system of the image. This allows tracked instruments to be located and appropriate iconic representations displayed relative not only to the landmark but also to the offset position location itself which is referenced to the landmark.

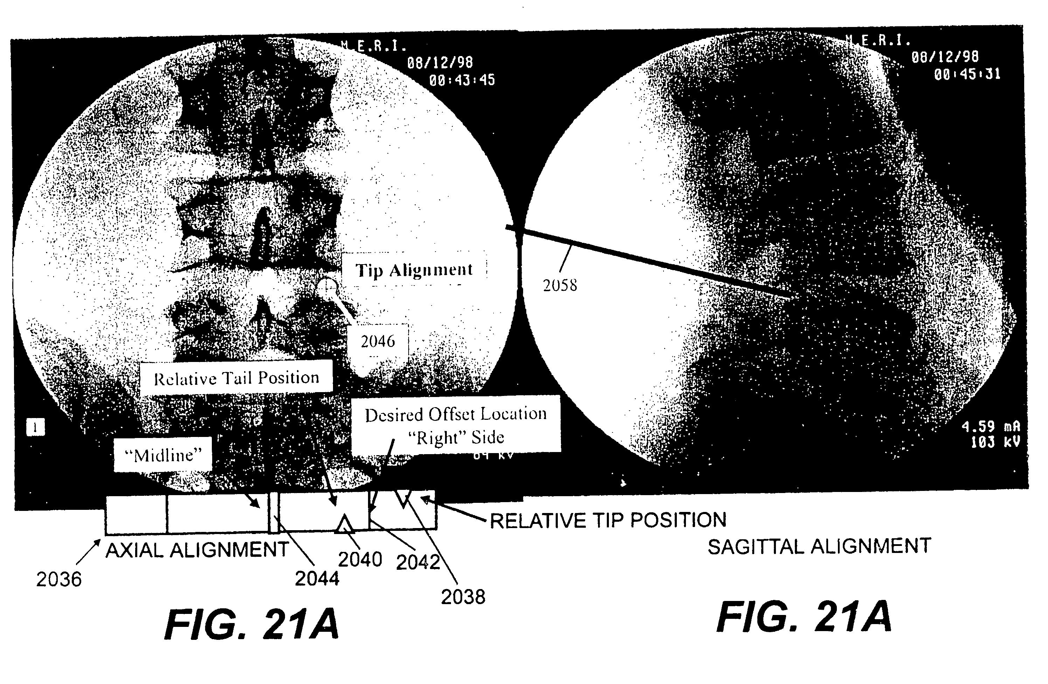

In the present apparatus 2000, further computer instructions are provided in system memory 2018 (or storage device 2026) to generate and display on monitor 121, a dual slider bar icon 2036 indicative of the positions of the tip and tail of the tracked instrument relative to the desired offset position. As shown e.g. in Figs. 21A, slider bar icon 2036 presents iconic representations of the relative positions of both the tip 2038 (upper track), and the instrument tail 2040 (lower track) of distractor 2050 (See Fig. 20A) with respect to the offset position representation 2042. Slider bar icon 2036 provides a convenient, real time assessment of the relative positions while the surgeon is moving the instrument. Icon 2036 preferably presents the relative positions in enlarged or magnified scope for ease of viewing and increased accuracy, and also can include a midline plane representation 2044. The additional instructions in system memory 2018 (or in storage device 2026) also can include instructions to generate and display in registration with the image, icons corresponding to one or both of the tip and tail (icon 2046 shown in Fig. 21A represents the instrument tip). Of course, icons other than a slider bar-type icon can be used in accordance with the present invention to display the relative position information need for the placement procedure.

Although not shown, the instructions in system memory 2018 (or in storage device 2026) alternatively can provide for the generation and display of iconic representations of the instrument or representative parts, such as the tip and tail, and of the offset position itself, in registration with the displayed image and the displayed landmark. One skilled in the art would understand, for example, that when the representations of the tip and tail of the instrument were coincident with the offset position, the surgeon is assured of both the correct positioning and alignment of the instrument relative to the offset position and the landmark, such as a midline plane.

### Interbody Fusion Device Placement Procedure

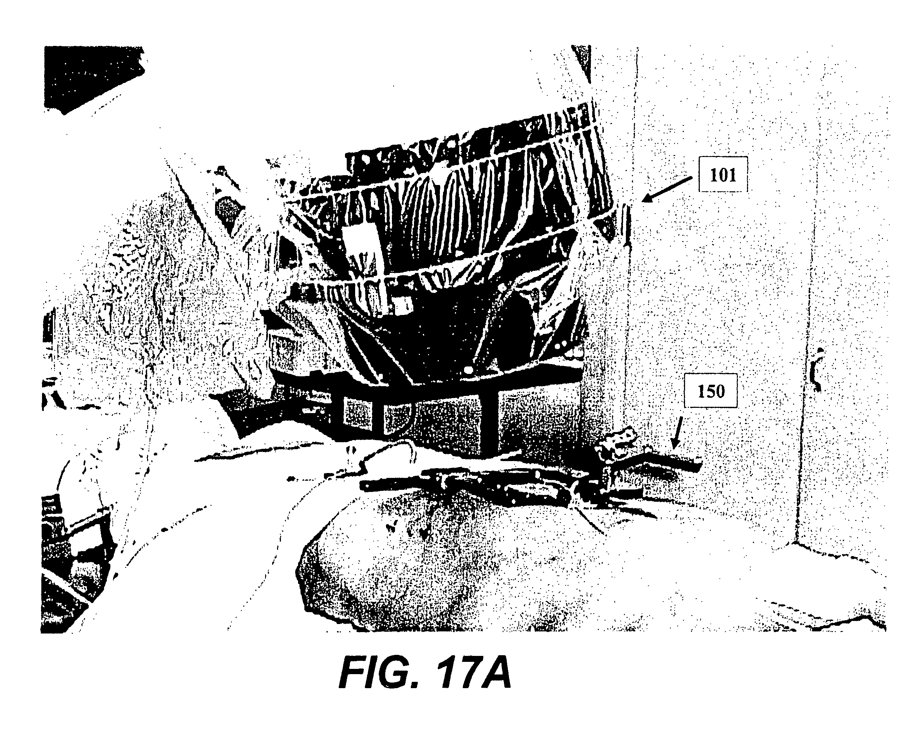

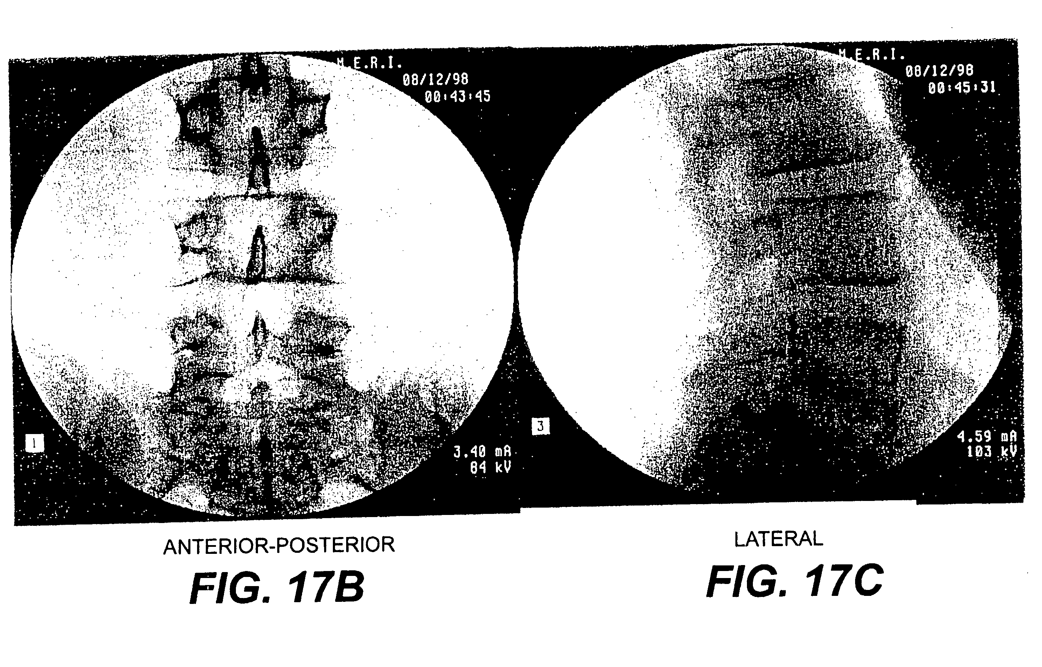

In accordance with a method consistent with the present invention, an appropriate anatomical image of the anatomy to be operated upon is first caused to be displayed on a display device. As embodied herein and with reference to the figures, Figs. 17B and 17C show, respectively, anterior-posterior ("A-P") and lateral X-ray images obtained using the apparatus shown in 17A. The apparatus shown in 17A includes apparatus 2000 in use with the image guided surgical system 100, as depicted schematically in Fig. 1, including fluoroscopic device 101 used for obtaining the x-ray images which can be processed by the image guided surgical system to provide accurate A-P and lateral images. The Fig. 17A apparatus also includes a dynamic reference array 150 attached to the spine of the patient which can be sensed by sensor 130 (not shown in Fig. 17A). The image guided surgical system "knows" the relative location of the reference array 150 and the fluoroscope device 101, and also has the ability to calibrate the images and "learn" the characteristics of the image such that it can overlay an icon representing the position of tracked surgical instruments onto the calibrated images shown in Figs. 17B and 17C during the actual fusion device placement procedure.

Further, in accordance with the method consistent with the present invention, the one or more anatomical landmark positions are inputted into the image guided surgical system computer. As embodied herein, this step can be accomplished by the surgeon in one or the other of two basic ways, namely using input device 2010 (or using the system's input device via appropriate instructions in memory 2018 or storage device 2026), or using a tracked surgical instrument like probe 2020. In the first alternative, the surgeon can input the coordinates of the landmark positions into the computer directly, such as using a conventional keyboard, mouse, or light pen input device that is suitably interfaced with the image guided surgery system computer 120. Fig. 18A is the A-P image corresponding to Fig. 17B with the points 2012 and 2014 selected and inputted by the surgeon superimposed. Fig. 19A shows the alternative route to inputting the landmark positions, namely the surgeon using tracked surgical probe 2020 in place of, or as part of, input device 2010 and physically touching the patient's anatomy at the locations that he has selected as corresponding to the desired anatomical landmark, in this case a midline plane.

Still further in accordance with the present invention, the method includes the step of processing the inputted anatomical landmark positions using additional computer instructions that, when executed cause the processor to generate and display a corresponding anatomical landmark on the display device and registration with the displayed anatomical image. As embodied herein, using the inputted landmark positions such as points 2012 and 2014, computer 120 in each case then would generate midline plane 2016 that passes through the two points and is parallel to the imaging direction when the A-P image was acquired (i.e., perpendicular to the plane of the A-P image).

The next step in the placement procedure is for the surgeon to select and input an offset position corresponding to the desired placement position and orientation for the interbody fusion device. The disclosed system allows the surgeon to input the desired offset from the midline plane such as by keyboard-type input device 2010 or to accept default values based on the diameter of the interbody device. In this latter respect, the offset position values, such as those suggested by the manufacturer, can be previously stored in memory 2018 along with appropriate additional instructions to select an appropriate value based on an input of the size/diameter of the interbody fusion device actually being used by the surgeon. Alternatively, the offset position values can be stored along with the additional instructions in storage device 2026.

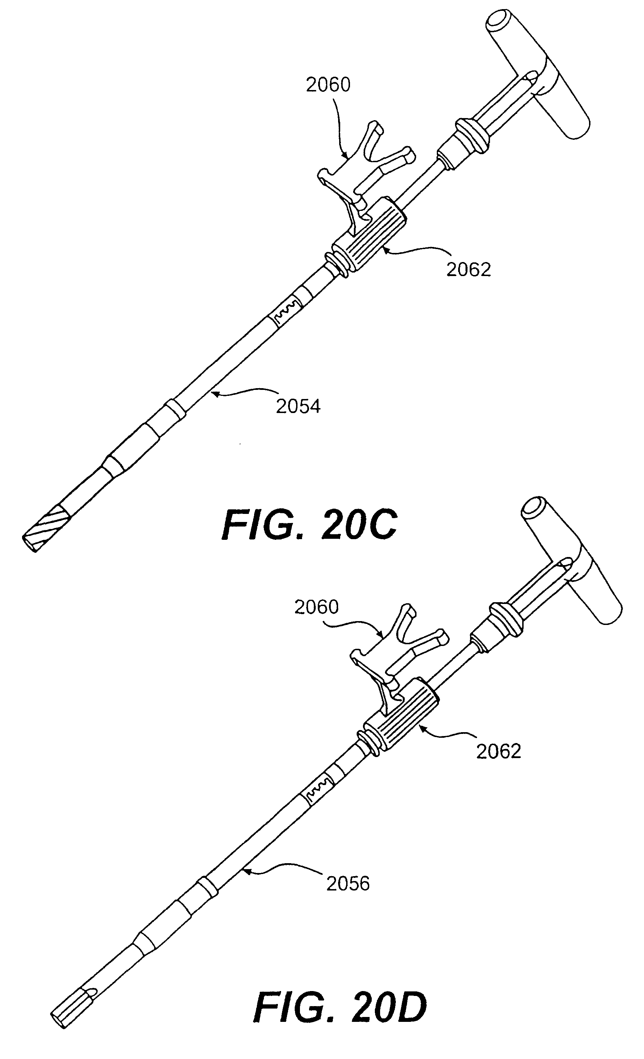

The next step in the placement procedure device positioning a distractor, such as distractor 2050 shown in Fig. 20A, to allow placement of the working channel instrument 2052 (Fig. 20B) that will be used to automatically guide the other instruments used to prepare the cavity and insert the actual interbody fusion device 2032, namely reamer 2054 shown in Fig. 20C, tap 2056 shown in Fig. 20D, and implant inserter 2034 shown in Fig. 20E.

Specifically, Figs. 21A and 21B are the respective A-P and lateral views showing the surgeon's first attempt to bring distractor 2050 into the correct position and alignment with the previously established offset position. Because of the tracking ability of the image guided surgery system, the position of the distractor (represented by tip icon 2046) can be displayed on the A-P view (Fig. 21A) and line icon 2058 representing the trajectory and insertion depth of distractor 2050 can be displayed on the lateral view in Fig. 21 B. Both the positional and trajectory/orientation representations of the distractor, of course, are in registration with the respective images.

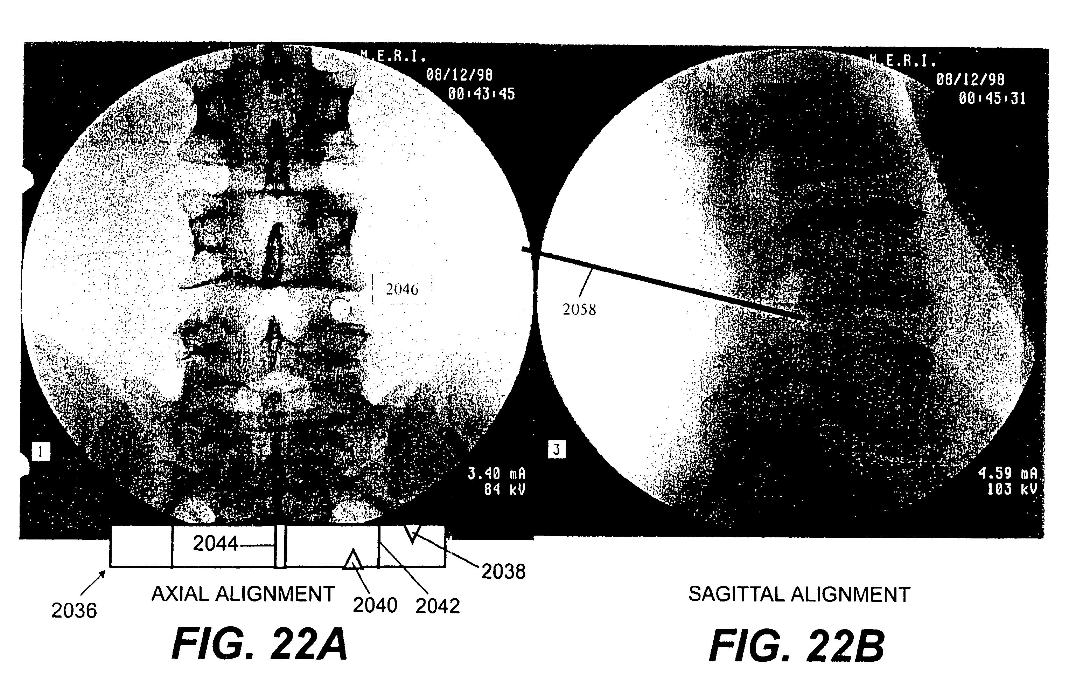

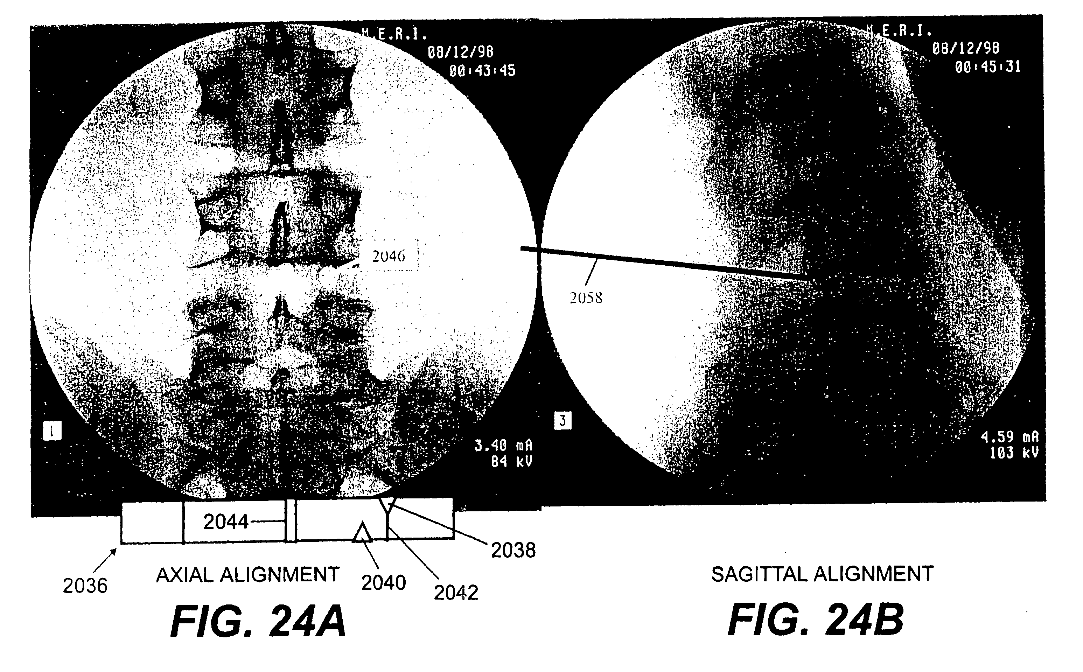

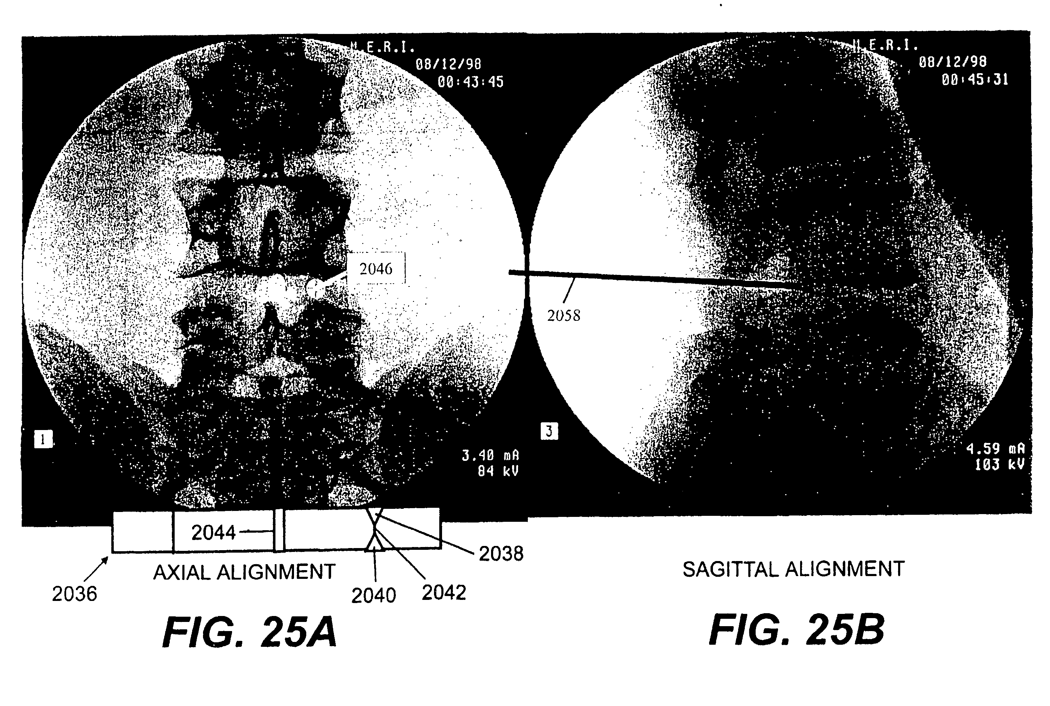

As shown in the Fig. 21A, the present invention provides computer instructions for the generation and displaying the dual track slider bar icon 2036 for allowing the surgeon to quickly adjust the position and orientation of the distractor to achieve the correct position and alignment relative to the desired offset position. As seen in Fig. 21A, at the beginning of this step the iconic representations of both the tip position 2038 and tail position 2040 of the distractor are not coincident with the selected offset position, as represented by the bar element 2044. Figs. 22A and 22B, through 25A and 25B represent relative positions during movement by the surgeon of the distractor to achieve positional and orientation alignment with the desired offset location during successive time periods. Note, for example, that in Fig. 24A the icon tip position 2038 alignment has been achieved with icon offset position 2042 but that the icon tail position 2040 alignment has yet to be achieved. This would indicate the situation where the tip of the distractor is at the desired offset position in the A-P frame of reference but that the axial orientation of the distractor is not yet aligned. Fig. 25A shows both tip and tail aligned coincident with offset position and parallel to the midline plane.

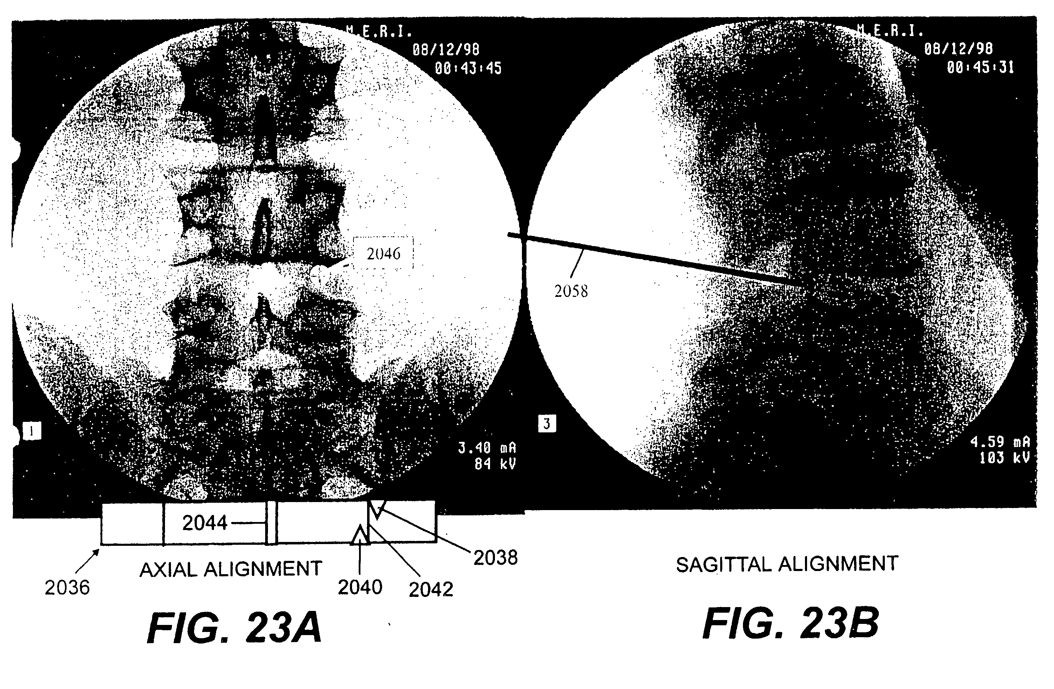

The respective lateral views in Figs. 21 B through 25B shows that both tip depth and trajectory in the sagittal plane are displayed. This can be achieved as a consequence of the dimensions of the various instruments, including distractor 2050, being known such that the locations of all points on the distractor can be computed and displayed based on the sensor sensing the LED or reflector array 2060 attached to the distractor. Note that in the instruments depicted in Figs. 20A, and Figs. 20C through 20E, the LED array 2060 is part of a universal attachment-type device 2062 to mount various working elements (i.e., a distractor element, an awl/reamer element, a tap element, and an inserter element). See U.S. Patent Application S.N. 08/971,126, of Kevin T. Foley et al., filed November 20, 1997 entitled "An Image Guided Awl/Tap/Screwdriver" for a description of a suitable universal attachment device. See also, U.S. Patent Application No. 09/296,251 (attorney docket no. 06148.0086-00000) of Thomas R. Williams, filed concurrently, entitled "Image Guided Universal Tool Adaptor and Method for Use With Computer Assisted Image Guided Surgery," and U.S. Patent Application No. 08/209,248, filed December 10, 1998, entitled "Image Guided Spinal Surgery Guide System and Method for Use Thereof."

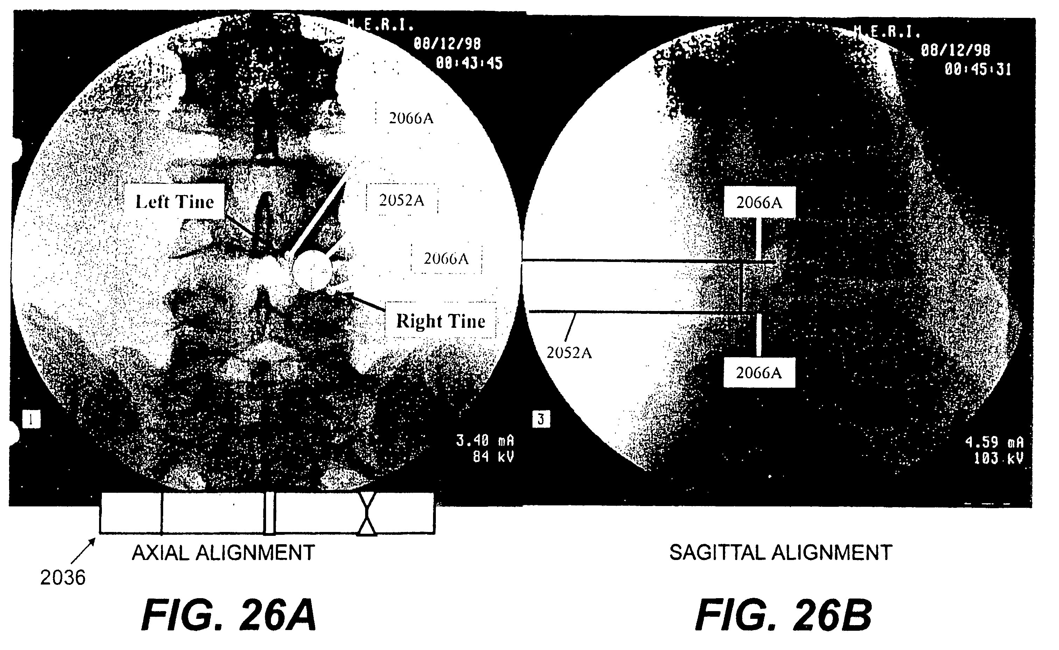

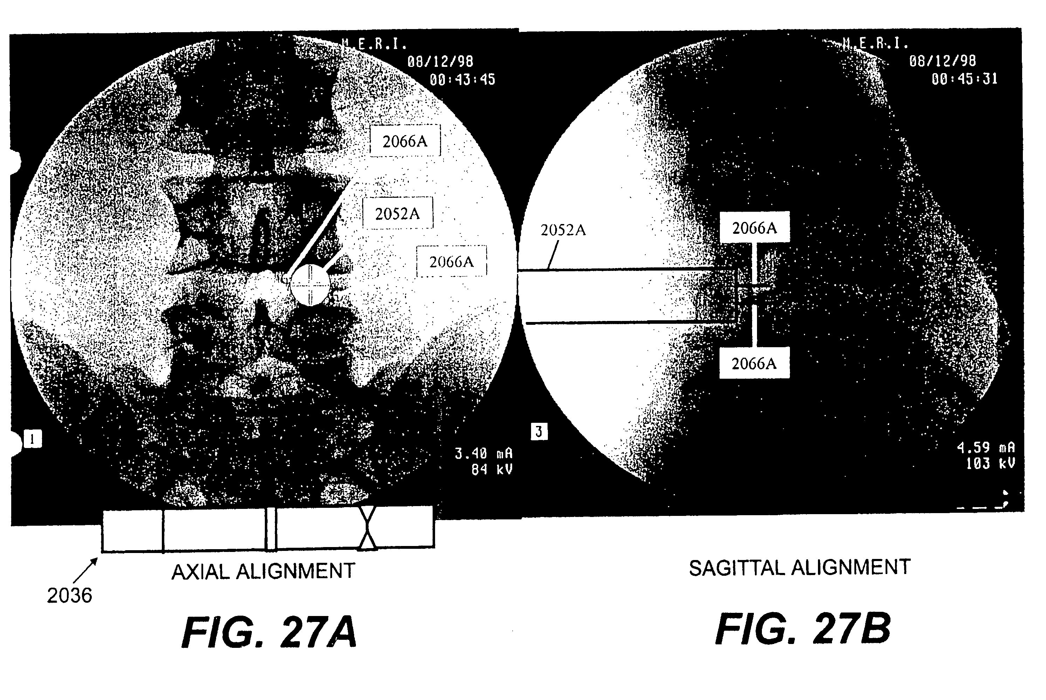

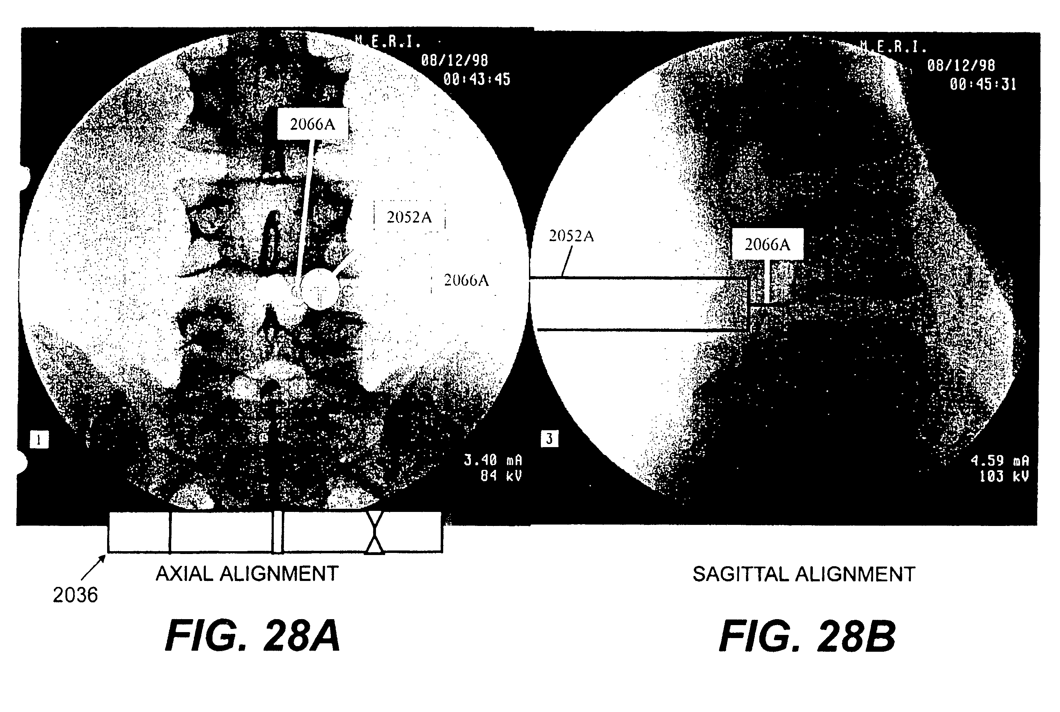

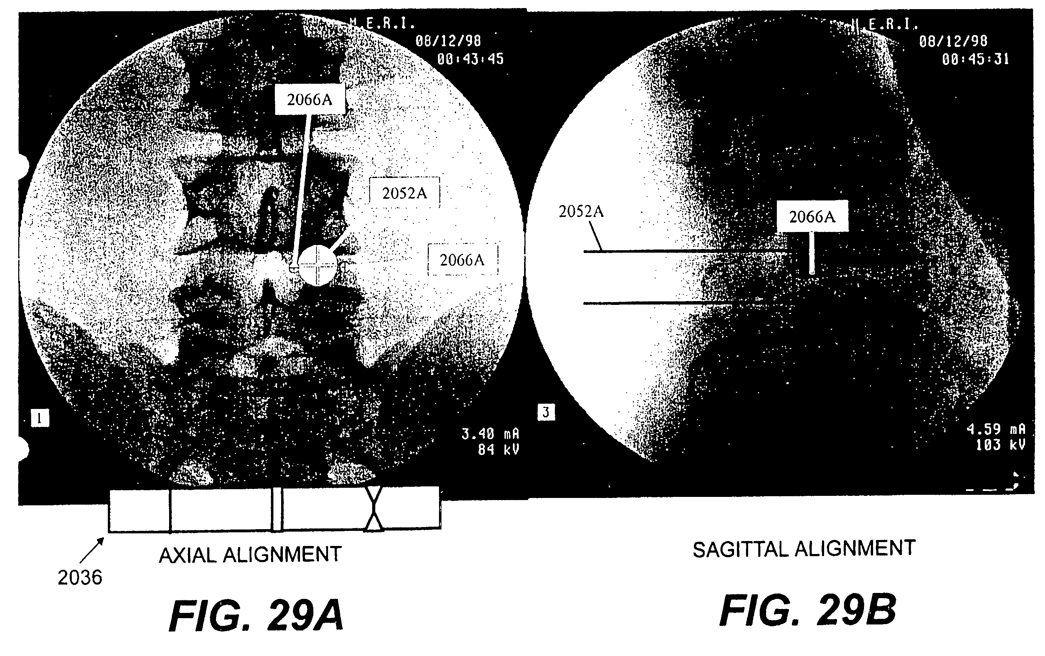

The next step in the placement procedure is the installation of the working channel such as working channel 2052 shown in Fig. 20B. Working channel 2052 is intended to fit over the distractor and has its own LED or reflector tracking array 2064. The particular working channel 2052 shown in Fig. 20B has a pair of tines 2066 radially spaced from the working channel axis 2068 to maintain the distraction provided by the distractor. The image guided surgical system 100 can compute and display iconic representations of not only the working channel (cylinder 2052A), but also the tines (cross hairs 2066A) to provide the desired rotational orientation of the working channel as it is entered into the disk space (i.e., tines 2066 should be parallel to the end plates of the vertebral bodies). Figs. 26A and 26B through 29A and 29B are a series of A-P and lateral x-ray images showing successive times during the working channel placement step where the surgeon gradually rotates the working channel until the positions of the tines are in the desired position parallel to the vertebral end plates, as in Figs. 28A and 28B. Figs. 29A and 29B represent the subsequent insertion of the working channel 2052 to the correct depth, as determined principally by the lateral view in Fig. 29B.

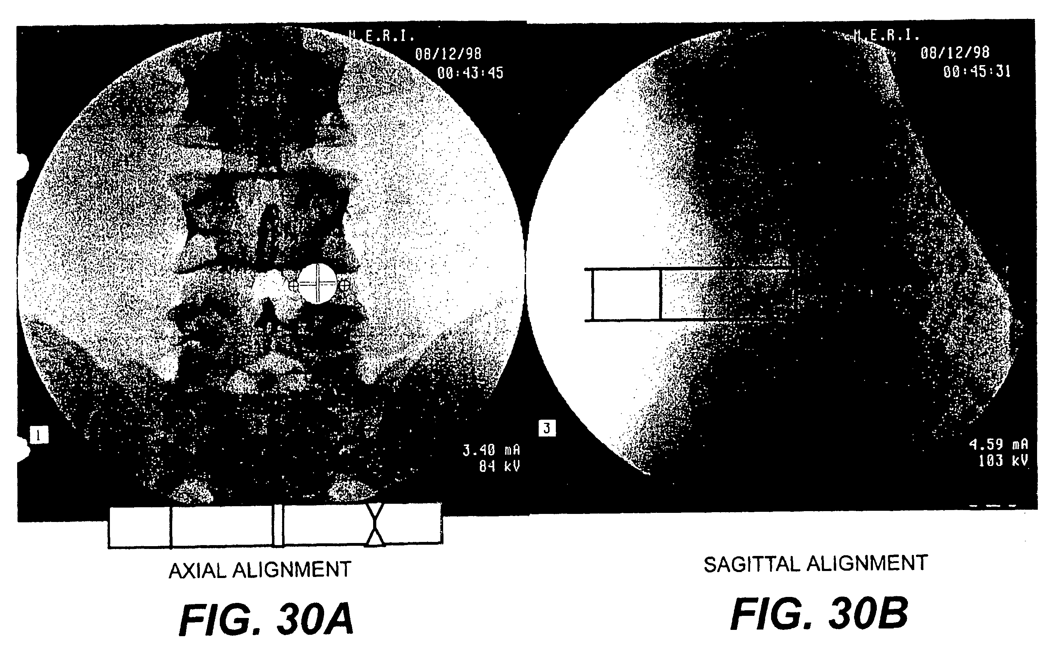

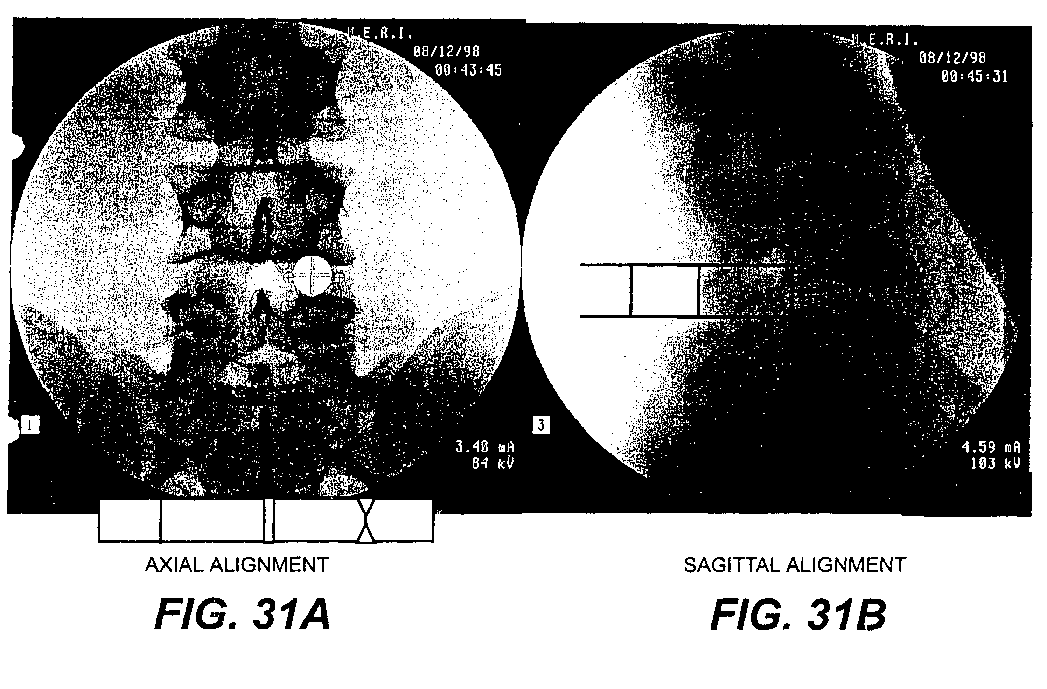

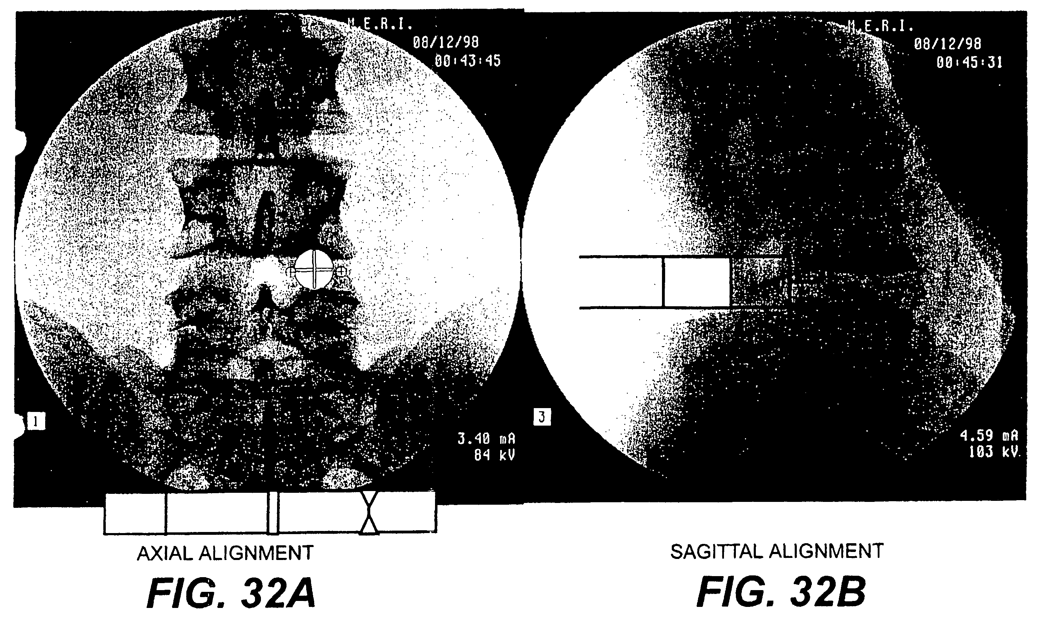

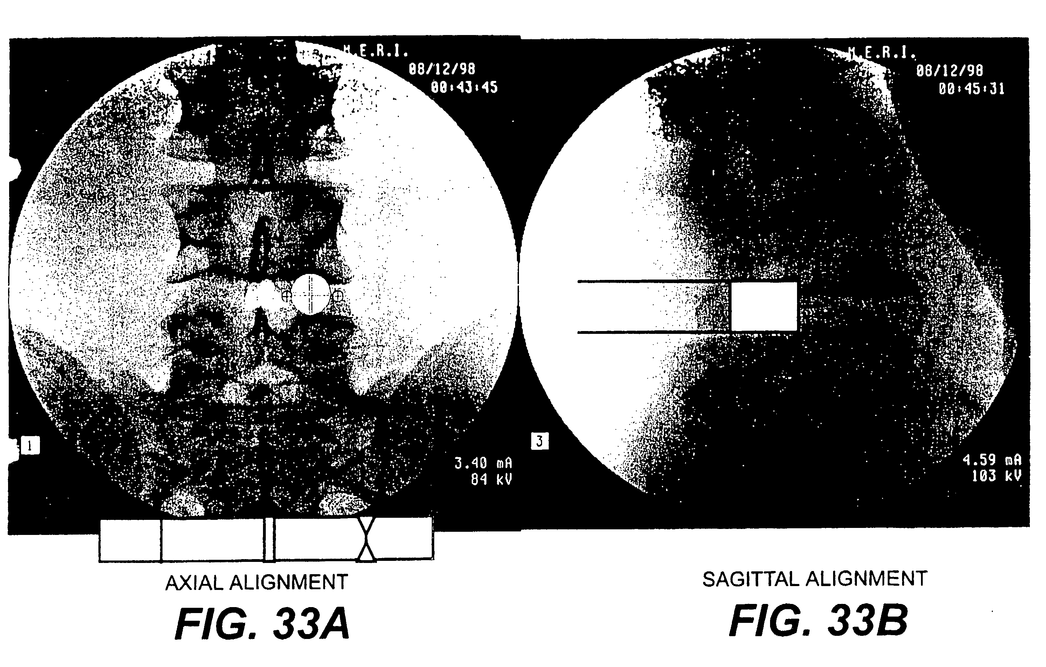

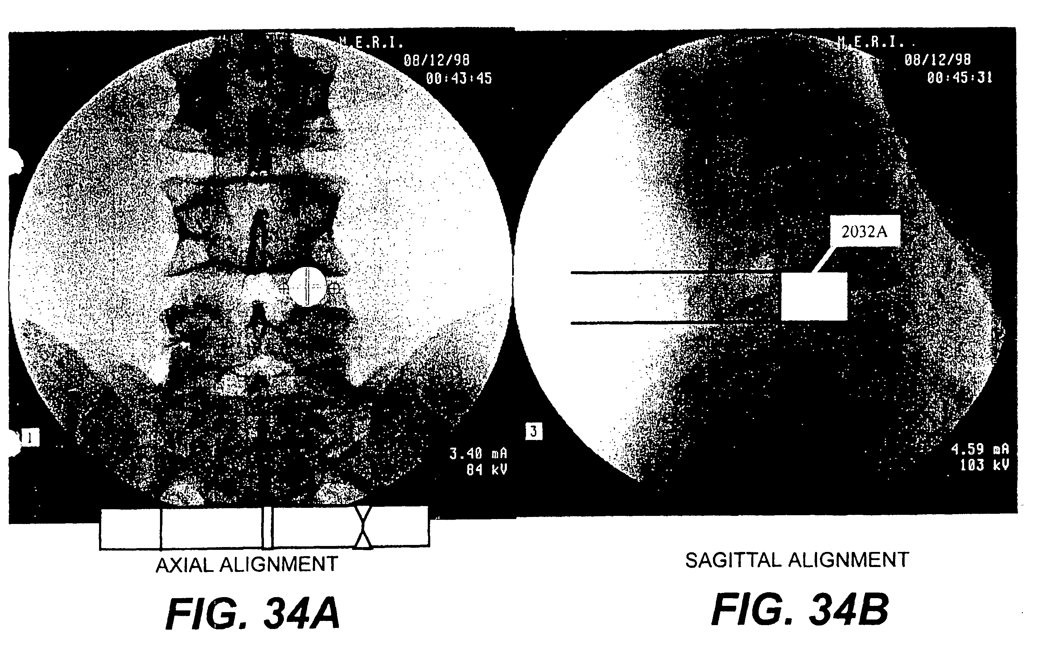

Figs. 30A and 30B through 34A and 34B represent the subsequent steps of reaming, tapping, and placing the interbody fusion device, such as through the use of the instruments shown in 20C, 20D, and 20E operating through the in-place working channel 2052 shown in Fig. 20B from which distractor 2050 has been removed. Note that the respective lateral views in these figures provide the surgeon with an accurate indication of the depth of not only the reamer and tap instruments, but also of the final placement of the interbody fusion device (iconically represented by cylinder 2032A in Fig. 34B). This latter aspect, of course, is a consequence of computer 120 "knowing" the location of all points on implant inserter 2034, including the attached interbody fusion device 2032, as a consequence of the known dimensions of the inserter and interbody fusion device itself. Like the offset position values, the dimensions the interbody fusion devices could be stored in memory and available to the computer such that, once selected/designated by the surgeon via input device 2010 or an input device provided with system 100, the required dimensions would be available to the computer 120 to generate the required coordinate transformations to allow the locations of key points on the instrument and interbody fusion device, *e.g.*, the tip and the tail, to be generated and iconic representations displayed.

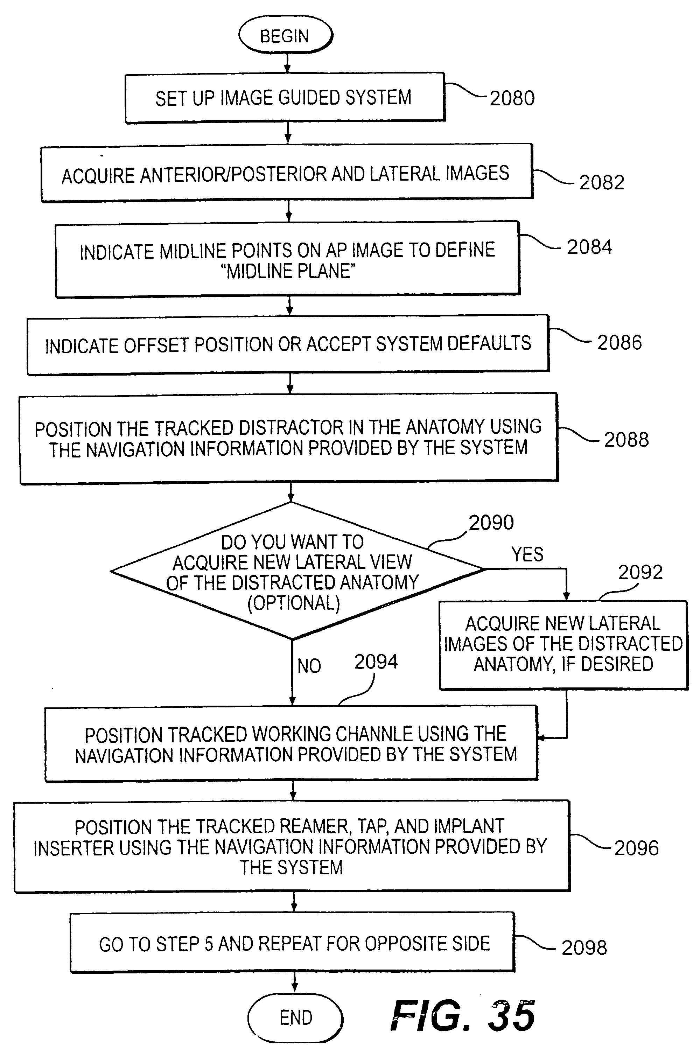

Fig. 35 is a flow chart summarizing the steps of an interbody fusion device placement procedure in accordance with the present invention. In particular, steps 2080 and 2082 entail, respectively, setting up the image guided system 100 and acquiring and displaying on monitor 121 the A-P and lateral views of the spinal portion of interest. As stated previously, pre-acquired images stored in memory 2018 can be used.

Next, step 2084 is carried out by inputting the coordinates of the midline via input device 2010 or using a tracked instrument like touch-probe 2020. Following generation of the midline plane by computer 120 and display on monitor 121 in registration with the acquired A-P and lateral images, step 2086 is accomplished by either inputting the offset position coordinates, such as by using input device 2010, or by accepting default values stored in memory 2018 (or provided in storage device 2026).

Next, in step 2088, the surgeon positions a tracked distractor, such as distractor 2050 shown in Fig. 20A, at the offset position using the image guided system 100 and the displayed offset position and slider bar icon 2036. One or more new lateral images of the distracted anatomy can optionally be acquired and displayed at this time by system 100, via steps 2090 and 2092, for subsequent use in positioning the working channel.

In step 2094, a tracked working channel, such as device 2052 (Fig. 20B), is positioned in the anatomy at the offset position using system 100 and the displayed offset position and slider bar icon 2036. The working channel positioning step 2094 includes x, y, z positioning, angular, and rotational orientation positioning of the working channel, as discussed previously.

Next, final step 2096 resulting in placement of the interbody fusion device is carried out using image guided system 100. Step 2096 includes substeps of consecutively positioning a tracked reamer, tap, and inserter, such as the instruments shown in Figs. 20C-20E.

Finally, step 2098 includes repeating steps 2086 through 2096 for placement of a fusion device at a corresponding offset position on the opposite side of the midline plane, as the devices are usually installed in pairs. Of course, if both right and left offset positions were inputted during step 2086, then only steps 2088 through 2096 need to be repeated as comprising step 2098.

The above described apparatus and method significantly extend the capabilities and advantages of image guided surgical systems, including image guided surgical systems used for image guided interbody fusion device placement. It would be apparent to those skilled in the art that various modifications and variations can be made to the present invention without departing from the scope or spirit of the invention. As stated previously, the advantages and benefits of the present invention are not confined to interbody fusion device placement surgical procedures although it is of particular benefit to that surgical procedure.

Also, although aspects of the present invention are described as being stored in memory, one skilled in the art will appreciate that these aspects can also be stored on other types of computer-readable media, such as secondary storage devices, like hard disks, floppy disks, or CD-ROM; a carrier wave from the Internet or other propagation medium; or other forms of RAM or ROM.

While this invention has been variously described generally in connection with LEDs and camera arrays, it should be recognized that other tracker elements and corresponding sensor arrays known in the art could be used, such as for example sonic, optic, or electromagnetic, as well as optical reflectors and a corresponding camera system.

Other embodiments of the invention will be apparent to those skilled in the art from consideration of the specification and practice of the invention disclosed herein.

## Claims

1. Apparatus (2000) for use in an interbody fusion device placement surgical procedure, the apparatus for use with an image guided surgical system (100), the system including a computer processor (120), a display device (121) coupled to the processor, and a memory (2018, 2026) associated with the processor and having stored computer instructions that, when executed, cause the processor to display on the display device at least an A-P image or a lateral image corresponding to the portion of a spine on which the placement procedure is'to be performed (2082), the apparatus **characterised by** comprising:
an interface (2010, 2020) for inputting one or more positions (2012, 2018) to the processor and for generating a midline plane landmark (2016);
additional computer instructions stored in the memory that, when executed, cause the processor to generate and display on the display device in registration with at least one of the displayed A-P and lateral images, a midline plane (2016) corresponding to said one or more positions; and
further instructions for generating offset implantation positions relative to said midline plane based on inputted offset position values.

2. The apparatus as claimed in claim 1 wherein the memory is associated with the processor.

3. The apparatus as in claim 1 or 2, wherein the interface is selected from the group consisting of a keyboard, a mouse, and a light pen.

4. The apparatus as in claim 1, 2 or 3, wherein said interface includes a surgical instrument.

5. The apparatus as in any one of claims 1 to 4 wherein said additional computer instructions include further instructions that, when executed, cause the computer processor to generate and display one or more icons associated with anatomical landmark positions.

6. The apparatus as in any one of claims 1 to 5 wherein the image guided surgical system has one or more trackable instruments, and wherein an icon indicates the position and orientation of a tracked instrument relative to an anatomical landmark or a location referenced to the landmark.

7. The apparatus as in claims 6 wherein the instrument has a tip portion and a tail portion, and wherein said icon indicates both the tip portion and tail portion positions relative to said anatomical landmark or a location referenced to the landmark.

8. The apparatus as in claim 6 or 7 wherein the instrument has a portion radially spaced from an instrument axis, and wherein said icon indicates the radial portion angular orientation relative to said anatomical landmark or a location referenced to the landmark.

9. The apparatus as in any one of claims 1 to 8 wherein the landmark is a plane.

10. The apparatus as in claim 1 wherein the additional instructions include instructions for generating and displaying an icon indicating the position and orientation of a tracked instrument relative to said plane or a location referenced to the plane.

11. The apparatus as in claim 10 wherein said icon is a slider bar icon (2306).

12. The apparatus as in claim 10 wherein the tracked instrument has a tip and a tail, and wherein said icon indicates the positions of the tip (2038) and the tail (2040) relative to said plane or to a location referenced to the plane.

13. The apparatus as in any one of claims 1 to 12 wherein said offset positions are stored in the memory and correspond to interbody fusion device manufacturers recommended placement positions.

14. The apparatus as in any one of claims 4 to 13 wherein the interface is a trackable surgical probe (2020).

15. The apparatus as in claim 1 further including one or more trackable surgical instruments, wherein said memory contains further instructions that, when executed, cause the processor to generate and display an icon representative of the position and orientation of a said trackable instrument relative to the midline plane or an offset position location referenced to the midline plane.

16. The apparatus as in claim 14 wherein said surgical instrument is a working channel instrument (2052) that defines a channel to automatically guide other instruments used to prepare a cavity and insert the interbody fusion device.

17. The apparatus as in claim 16 wherein the other instruments include a reamer (2054), a tap (2056), and an implant inserter (2034).

18. The apparatus as in claim 16 wherein the working channel instrument includes a pair of tines (2066) that are positioned parallel to end plates of vertebral bodies.

19. The apparatus as in any one of claims 1 to 18 wherein the computer memory includes stored offset position values, and the computer instructions include further instructions that, when executed, cause the computer processor to default to a stored offset position and generate an offset position referenced to the midline plane based on the default value.

20. The apparatus as in any one of claims 4 to 18 wherein the tracked instrument (140) has a tip (602) and a tail (603), and wherein the icon indicates the positions of both the tip (2038) and the tail (2040) relative to the offset position location.

21. The apparatus as in claim 8 wherein the icon indicates the positions of the tip and tail relative to the midline plane.

22. The apparatus as in claim 8 wherein the icon is a slider-bar.

23. The apparatus as in any one of claims 4 to 18 wherein the tracked instrument has a portion radially spaced from an instrument axis, and wherein the icon (108) indicates the rotational orientation of the radial portion relative to the offset position.

## Patentansprüche

1. Vorrichtung (2000) zur Verwendung in einer chirurgischen Prozedur für die Platzierung einer Zwischenkörperfusionseinrichtung, wobei die Vorrichtung zur Verwendung mit einem bildgeführten chirurgischen System (100) vorgesehen ist, wobei das System einschließt einen Computerprozessor (120), eine Anzeigeeinrichtung (121), die mit dem Prozessor gekoppelt ist, und einen Speicher (2018, 2026), der zu dem Prozessor gehört und in dem Computerbefehle gespeichert sind, die dann, wenn sie ausgeführt werden, den Prozessor veranlassen auf der Anzeigeeinrichtung wenigstens ein A-P Bild oder ein laterales Bild entsprechend zu dem Abschnitt einer Wirbelsäule, auf der die Platzierungsprozedur ausgeführt werden soll (2082), anzuzeigen, wobei die Vorrichtung **dadurch gekennzeichnet ist, dass** sie umfasst:
eine Schnittstelle (2010, 2020) zum Eingeben von ein oder mehreren Positionen (2012, 2018) in den Prozessor und zum Erzeugen einer Mittellinienebenen-Landmarke (2016);
in dem Speicher gespeicherte zusätzliche Computerbefehle, die dann, wenn sie ausgeführt werden, den Prozessor veranlassen eine Mittellinienebene (2016) entsprechend zu der einen oder den mehreren Positionen zu erzeugen und diese auf der Anzeigeeinrichtung ausgerichtet zu dem angezeigten A-P und/oder dem lateralen Bild anzuzeigen; und
weitere Befehle zum Erzeugen von Versatz-Implantationspositionen relativ zu der Mittellinienebene auf Grundlage von eingegebenen Versatz-Positionswerten.

2. Vorrichtung nach Anspruch 1, wobei der Speicher zu dem Prozessor gehört.

3. Vorrichtung nach Anspruch 1 oder 2, wobei die Schnittstelle aus der Gruppe gewählt ist, die aus einer Tastatur, einer Maus und einem Lichtstift besteht.

4. Vorrichtung nach Anspruch 1, 2 oder 3, wobei die Schnittstelle ein chirurgisches Instrument einschließt.

5. Vorrichtung nach irgendeinem der Ansprüche 1 bis 4, wobei die zusätzlichen Computerbefehle weitere Befehle einschließen, die dann, wenn sie ausgeführt werden, den Prozessor veranlassen ein oder mehrere Icons, die zu anatomischen Landmarkenpositionen gehören, zu erzeugen und anzuzeigen.

6. Vorrichtung nach irgendeinem der Ansprüche 1 bis 5, wobei das bildgeführte chirurgische System ein oder mehrere verfolgbare Instrumente aufweist, und wobei ein Icon die Position und Orientierung eines verfolgten Instruments relativ zu einer anatomischen Landmarke oder einem Ort in Bezug auf die Landmarke anzeigt.

7. Vorrichtung nach Anspruch 6, wobei das Instrument einen Spitzen-Abschnitt und einen End-Abschnitt aufweist, und wobei das Icon von Positionen sowohl des Spitzen-Abschnitts als auch des End-Abschnitts relativ zu der anatomischen Landmarke oder einem Ort in Bezug auf die Landmarke anzeigt.

8. Vorrichtung nach Anspruch 6 oder 7, wobei das Instrument einen Abschnitt aufweist, der radial von einer Instrumentenachse beabstandet ist, und wobei das Icon die Winkelorientierung des radialen Abschnitts relativ zu der anatomischen Landmarke oder einem Ort in Bezug auf die Landmarke anzeigt.

9. Vorrichtung nach irgendeinem der Ansprüche 1 bis 8, wobei die Landmarke eine Ebene ist.

10. Vorrichtung nach Anspruch 1, wobei die zusätzlichen Befehle Befehle zum Erzeugen und Anzeigen eines Icons, das die Position und Orientierung eines verfolgten Instruments relativ zu der Ebene oder einem Ort in Bezug auf die Ebene anzeigt, einschließen.

11. Vorrichtung nach Anspruch 10, wobei das Icon ein Schiebebalken-Icon (2306) ist.

12. Vorrichtung nach Anspruch 10, wobei das verfolgte Instrument eine Spitze und ein Ende aufweist, wobei das Icon die Positionen der Spitze (2038) und des Endes (2040) relativ zu der Ebene oder zu dem Ort in Bezug auf die Ebene anzeigt.

13. Vorrichtung nach irgendeinem der Ansprüche 1 bis 12, wobei die Versatz-Positionen in dem Speicher gespeichert werden und empfohlenen Platzierungspositionen von Herstellern einer Zwischenkörperfusionseinrichtung entsprechen.

14. Vorrichtung nach irgendeinem der Ansprüche 4 bis 13, wobei die Schnittstelle eine verfolgbare chirurgische Sonde (2020) ist.

15. Vorrichtung nach Anspruch 1, ferner umfassend ein oder mehrere verfolgbare chirurgische Instrumente, wobei der Speicher weitere Befehle enthält, die dann, wenn sie ausgeführt werden, den Prozessor veranlassen ein Icon, das die Position und Orientierung eines besagten verfolgbaren Instruments relativ zu der Mittellinienebene oder einem Versatz-Positionsort in Bezug auf die Mittellinienebene darstellt, zu erzeugen und dieses anzuzeigen.

16. Vorrichtung nach Anspruch 14, wobei das chirurgische Instrument ein Arbeitskanalinstrument (2052) ist, das einen Kanal definiert, um andere Instrumente automatisch zu führen, die verwendet werden, um einen Hohlraum vorzubereiten und die Zwischenkörperfusionseinrichtung einzuführen.

17. Vorrichtung nach Anspruch 16, wobei die anderen Instrumente eine Reibahle (2054), einen Bohrer (2056), und einen Implantat-Einfüger (2034) umfassen.

18. Vorrichtung nach Anspruch 16, wobei das Arbeitskanal-Instrument ein Paar von Zacken (2066) umfasst, die parallel zu Endplatten von Wirbelkörpern positioniert sind.

19. Vorrichtung nach irgendeinem der Ansprüche 1 bis 18, wobei der Computerspeicher gespeicherte Versatz-Positionswerte einschließt, und die Computerbefehle ferner Befehle einschließen, die dann, wenn sie ausgeführt werden, den Computerprozessor veranlassen eine Voreinstellung auf eine gespeicherte Versatzposition vorzunehmen und eine Versatzposition in Bezug auf die Mittellinienebene auf Grundlage des voreingestellten Werts zu erzeugen.

20. Vorrichtung nach irgendeinem der Ansprüche 4 bis 18, wobei das verfolgte Instrument (140) eine Spitze (602) und ein Ende (603) aufweist, und wobei das Icon die Positionen sowohl der Spitze (2038) als auch des Endes (2040) relativ zu dem Versatz-Positionsort anzeigt.

21. Vorrichtung nach Anspruch 8, wobei das Icon die Positionen der Spitze und des Endes relativ zu der Mittellinienebene anzeigt.

22. Vorrichtung nach Anspruch 8, wobei das Icon ein Schieberstab ist.

23. Vorrichtung nach irgendeinem der Ansprüche 4 bis 18, wobei das verfolgte Instrument einen Abschnitt aufweist, der radial von einer Instrumentenachse beabstandet ist, und wobei das Icon (108) die rotationsmäßige Orientierung des radialen Abschnitts relativ zu der Versatzposition anzeigt.

## Revendications

1. Appareil (2000) pour une utilisation dans une procédure chirurgicale de placement d'un dispositif de fusion entre corps, l'appareil étant destiné à une utilisation avec un système chirurgical guidé par imagerie (100), le système incluant un processeur informatique (120), un dispositif d'affichage (121) couplé au processeur et une mémoire (2018, 2026) associée au processeur et contenant des instructions informatiques stockées qui, lorsqu'elles sont exécutées, entraînent le processeur à afficher sur le dispositif d'affichage au moins une image A-P ou une image latérale correspondant à la portion d'une colonne vertébrale sur laquelle la procédure de placement doit être effectuée (2082), l'appareil étant **caractérisé par** le fait de comprendre:
une interface (2010, 2020) pour entrer une ou plusieurs positions (2012, 2018) dans le processeur et pour générer un repère dans le plan médian (2016);
des instructions informatiques supplémentaires stockées dans la mémoire qui, lorsqu'elles sont exécutées, entraînent le processeur à générer et à afficher sur le dispositif d'affichage, en inscription avec au moins une des images A-P et latérale affichées, un plan médian (2016) correspondant auxdites une ou plusieurs positions; et
d'autres instructions pour générer des positions d'implantation décalées relativement audit plan médian basées sur des valeurs de position décalées entrées.

2. Appareil suivant la revendication 1, dans lequel la mémoire est associée au processeur.

3. Appareil suivant la revendication 1 ou 2, dans lequel l'interface est choisie dans le groupe constitué d'un clavier, d'une souris et d'un stylo optique.

4. Appareil suivant la revendication 1, 2 ou 3, dans lequel ladite interface inclut un instrument chirurgical.

5. Appareil suivant l'une quelconque des revendications 1 à 4, dans lequel lesdites instructions informatiques supplémentaires incluent d'autres instructions qui, lorsqu'elles sont exécutées, entraînent le processeur informatique à générer et à afficher une ou plusieurs icônes associées à des positions de repères anatomiques.

6. Appareil suivant l'une quelconque des revendications 1 à 5, dans lequel le système chirurgical guidé par imagerie possède un ou plusieurs instruments pouvant être suivis et dans lequel une icône indique la position et l'orientation d'un instrument suivi relativement à un repère anatomique ou à une localisation référencée au repère.

7. Appareil suivant la revendication 6, dans lequel l'instrument possède une portion de pointe et une portion de queue et dans lequel ladite icône indique à la fois la position de la portion de pointe et la position de la portion de queue relativement audit repère anatomique ou à une localisation référencée au repère.

8. Appareil suivant la revendication 6 ou 7, dans lequel l'instrument possède une portion espacée dans la direction radiale d'un axe de l'instrument et dans lequel ladite icône indique l'orientation angulaire de la portion radiale relativement audit repère anatomique ou à une localisation référencée au repère.

9. Appareil suivant l'une quelconque des revendications 1 à 8, dans lequel le repère est un plan.

10. Appareil suivant la revendication 1, dans lequel les instructions supplémentaires incluent des instructions pour générer et afficher une icône indiquant la position et l'orientation d'un instrument suivi relativement audit plan ou à une localisation référencée au plan.

11. Appareil suivant la revendication 10, dans lequel ladite icône est une icône à barre coulissante (2306).

12. Appareil suivant la revendication 10, dans lequel l'instrument suivi possède une pointe et une queue et dans lequel ladite icône indique les positions de la pointe (2038) et de la queue (2040) relativement audit plan ou à une localisation référencée au plan.

13. Appareil suivant l'une quelconque des revendications 1 à 12, dans lequel lesdites positions décalées sont stockées dans la mémoire et correspondent à des positions de placement recommandées par les fabricants de dispositifs de fusion entre corps.

14. Appareil suivant l'une quelconque des revendications 4 à 13, dans lequel l'interface est une sonde chirurgicale pouvant être suivie (2020).

15. Appareil suivant la revendication 1, incluant en outre un ou plusieurs instruments chirurgicaux pouvant être suivis, dans lequel ladite mémoire contient d'autres instructions qui, lorsqu'elles sont exécutées, entraînent le processeur à générer et à afficher une icône représentative de la position et de l'orientation dudit instrument pouvant être suivi relativement au plan médian ou à une localisation de position décalée référencée au plan médian.

16. Appareil suivant la revendication 14, dans lequel ledit instrument chirurgical est un instrument à canal de travail (2052) qui définit un canal pour guider automatiquement d'autres instruments employés pour préparer une cavité et insérer le dispositif de fusion entre corps.

17. Appareil suivant la revendication 16, dans lequel les autres instruments incluent un alésoir (2054), un dispositif de perçage (2056) et un dispositif d'insertion d'implant (2034).

18. Appareil suivant la revendication 16, dans lequel l'instrument à canal de travail inclut une paire de dents (2066) qui sont positionnées parallèles aux plateaux vertébraux de corps vertébraux.

19. Appareil suivant l'une quelconque des revendications 1 à 18, dans lequel la mémoire informatique inclut des valeurs de positions décalées stockées et les instructions informatiques incluent d'autres instructions qui, lorsqu'elles sont exécutées, entraînent le processeur informatique à choisir par défaut une position décalée stockée et à générer une position décalée référencée au plan médian sur la base de la valeur par défaut.

20. Appareil suivant l'une quelconque des revendications 4 à 18, dans lequel l'instrument suivi (140) possède une pointe (602) et une queue (603) et dans lequel l'icône indique les positions à la fois de la pointe (2038) et de la queue (2040) relativement à la localisation de la position décalée.

21. Appareil suivant la revendication 8, dans lequel l'icône indique les positions de la pointe et de la queue relativement au plan médian.

22. Appareil suivant la revendication 8, dans lequel l'icône est une barre coulissante.

23. Appareil suivant l'une quelconque des revendications 4 à 18, dans lequel l'instrument suivi possède une portion espacée dans la direction radiale d'un axe de l'instrument et dans lequel l'icône (108) indique l'orientation de rotation de la portion radiale relativement à la position décalée.
